# EUROPEAN PATENT APPLICATION

(11) **EP 2 363 470 A1**
(43) Date of publication of application: **07.09.2011**
(21) Application number: 09822114.6
(22) Date of filing: 23.10.2009
(51) Int. Cl.: C12N 15/09, A01K 67/027, C12N 5/10

(54) **METHOD FOR INTRODUCING MUTATED GENE, GENE HAVING MUTATION INTRODUCED THEREIN, CASSETTE FOR INTRODUCING MUTATION, VECTOR FOR INTRODUCING MUTATION, AND KNOCK-IN NON-HUMAN MAMMAL**

(30) Priority: 23.10.2008 JP 2008273446
(71) Applicant: Fukuoka University, Fukuoka-shi, Fukuoka 814-0180 (JP); National University Corporation Kumamoto University, Kumamoto-shi Kumamoto 860-8555 (JP)
(72) Inventor: HIROSE Shinichi, Fukuoka-shi Fukuoka 814-0180 (JP); DESHIMARU Masanobu, Fukuoka-shi Fukuoka 814-0180 (JP); ARAKI, Kimi, Kumamoto-shi Kumamoto 860-8555 (JP)
(74) Representative: Gille Hrabal Struck Neidlein Prop Roos
(86) International application number: PCT/JP2009/068729
(87) International publication number: WO 2010/047423

(57) **Abstract**

Disclosed is a method for introducing a mutation into a gene, which comprises the following steps: a homologous recombination step of carrying out the homologous recombination between a target gene into which the mutation is to be introduced and a target recombinant vector, thereby substituting an exon in the target gene into which the mutation is to be introduced by a target DNA sequence in the target recombinant vector; and a mutation introduction step of carrying out the specific recombination between the target DNA sequence in the resulting target recombinant gene and a mutation introduction cassette of a mutation introduction vector carrying a mutated DNA sequence containing a mutant exon by the intervening action of Cre recombinase to substitute the target DNA sequence by the mutated DNA in the mutation introduction cassette, thereby producing a mutation-introduced gene into which the mutant DNA sequence has been introduced. The method enables the production of a knock-in non-human mammalian animal, such as a knock-in mouse, which carries the mutation-introduced gene.

## Description

### TECHNICAL FIELD

The present invention relates to a method for introducing a mutant gene, a mutation-introduced gene carrying a mutation introduced therein, a mutation introduction cassette for introducing a mutation, a mutation introduction vector for introducing a mutation, and a knock-in non-human mammalian animal. More particularly, the present invention relates to a method for introducing a mutant gene, which enables an introduction of a mutant DNA of interest into a target gene in a precise and quick manner with a high probability, a mutation-introduced gene having a mutation introduced therein, a method for the production of the same, a mutation introduction cassette for introducing a mutation, and a mutation introduction vector as well as a knock-in non-human mammalian animal.

### BACKGROUND TECHNOLOGY

It is not too much to say that all functions of a living thing to maintain its life are controlled by the gene having a "blueprint" that preserves genetic information. All the genetic information sis controlled by a combination of base pairs composed of four bases of DNAs. In case where the combination of the base pairs would be injured for some reasons causing abnormality in genes, the genetic information could not work in a normal fashion resulting in outbreaks of various diseases and so on.

For instance, in order to explicate mechanisms of causes or onsets of human diseases, it is of great importance to investigate which gene would be involved in the expression of the cause or onset of a disease. It is impossible, however, to investigate the gene involved therein using the human body, and it is also ethically problematical to investigate it directly using human tissues.

It can now be noted herein that, as a result of advances in the analysis of as human genome, among others, the human genome has been found in common with experimental animals such as mice and the like in a majority of genomes, and it has biological functions alike to those of such animals. It is thus possible to investigate the functions of the human genes ansd explicate mechanisms of their biological functions by introducing genes of the experimental animsls involved in gene mutations and expressing them. As a matter of course, the results obtained by the animal experiments cannot be applied intact to the human being because the human biological functions are different from those of the animals, however, they could be applied to the human being if an endogenous gene of an experimental animal could be modified by a human gene. At this end, there have been produced various genetically altered model animals for the purpose to explicate the mechanisms of human diseases and so on.

As the genetically altered model animals, there may be mentioned, for example, knock-out animals such as knock-out mice, etc., in which a gene involved in a metabolic enzyme or receptor is deleted, and knock-in animals in which a foreign gene is introduced into an endogenous gene of an experimental animal including mice and so on. At the present time, there have been produced many genetically altered model animals in which genes involved in various diseases are altered.

The genetically knock-out animals are model animals in which an originally present particular gene is wholly or partially altered, such as broken, deleted or replaced, and the gene is suppressed to cause no expression of its original functions. The knock-out animals may be used as a very useful experimental system for investigating the mechanisms of genes themselves and gene products or evaluating the importance of the genes because its original particular gene is altered and no protein corresponding to the original particular gene is produced by mutation. It can be noted herein, however, that a majority of general genetic diseases are caused by substitution of an amino acid or the like, due to a point mutation, but not due to a loss of a whole gene. In this sense, knock-out animals are considered to be less appropriate as a model for investigating such genetic abnormality.

For general transgenic animals, a mutant gene is expressed under the control of a core promoter sequence having several kbs or a site on a chromosome into which a mutant gene is introduced cannot be predicted, therefore, there may be frequently occurred cases where a gene introduced is not expressed as expected. This is a great barrier for analysis of functions of genes by using such transgenic animals and for development of pathophysiological model animals. Although experiments using transgenic animals have some problems, such transgenic animals have now been applied to versatile research fields because they can be produced for a relatively short period of time, so that experimental results can be evaluated within short.

On the other hand, a knock-in animal carries a foreign mutant gene by which a normal gene originally inherent in the animal has been replaced, so that a mutated protein derived from the foreign gene is considered to be less affected by the normal protein originally present in the animal. Therefore, the experimental results regarding the foreign gene can be considered to reflect the functions of its foreign gene.

It can be further noted herein, however, that the knock-in animals have the advantage in securing the formation of a model animal of interest because the knock-in animals can be formed from ES cells because they can be produced from ES cells by utilizing genetic targeting techniaues and the foreign mutant gene introduced can be expressed exactly by an endogenous promoter.

In order to exactly mimic a pathological condition of a genetic disorder and certainly assess the influence of a spontaneous mutation thereon, the knock-in animals can be said to be more advantageous than the knock-out animals. Although the method for the production of knock-in animals is established, the issue resides in the fact that conventional techniques for producing them are laborious and require a long-lasting period of time, e.g., for 1.5 to two years, and a large amount of expenses to produce knock-in animals. The knock-in animals have so far been produced by replacing a genomic DNA of a target gene by an exogenous genomic DNA by homologous recombination between the target gene and a target recombinant vector. The method for the production of the knock-in animals, therefore, has the great disadvantage that a probability of the homologous recombination to produce the objective genomic DNA from the genomic DNA of the target gene is very low such as less than approximately 10⁻⁵. Requests have been asked, accordingly, for development of a method for the production of knock-in animals with brevity and for a short period of time.

Therefore, the present inventors have tried to develop a technique for producing knock-in animals easily and within short by focusing attention on a Cre-loxP system that is one of the most useful tools for manipulating cells or genes of mice and so on (see non-patent publication Nos. 1 - 6). The Cre-loxP system is the system that can cleave the DNA portion flanked by two loxP sites by mediation of an enzyme recombinase, i.e., Cre (cyclinization recombinase). As a DNA of a foreign gene or the like is inserted into a site of a genomic DNA, the foreign gene DNA flanked by two loxP sites of the Cre-loxP system can be deleted by the event of cleavage and cyclinization recombination of the loxP sequence by mediation of Cre recombinase.

In order to allow an efficient introduction of a foreign DNA sequence, the present inventors have devised modifications of a Cre-loxP system having the properties as described above in such a manner that one or both of the two loxP sequences flanking the DNA sequence is or are mutated so as for the Cre recombinase to make it unfeasible or difficult to recognize the lox sequence, and that the foreign DNA sequence site flanked by the two lox sequences can be inserted into another gene, without being deleted, by recombination (see non-patent publication Nos. 6 - 8 and patent publication Nos. 1 and 2). In other words, the Cre recombinase can cut or bind a spacer sequence in the middle of the lox sequence if a mutant lox sequence would be present in one or both of the double strands of the loxP sequence constituting a base pair. As a result, the foreign DNA sequence site flanked by the two lox sequences is recombined and inserted.

There have been presented reports regarding a trap vector into which a foreign gene including, for example, a reporter gene, such as a luminescent gene, etc., is inserted by utilizing the mutant loxP sequence as described above (see patent publication No. 1, etc.), and a technique for producing a knock-out animal with such a trap vector introduced therein (see patent publication No. 2, etc.). Further reports have been presented regarding techniques of stably inserting a foreign DNA introduced between lox66 sequence as mutant loxP sequence and loxP sequence of a donner vector into a gene by recombination between lox71 sequence as a mutant loxP sequence of an accepter vector and lox66 sequence as a mutant loxP sequence of a donner vector or between the loxP sequences of the accepter vector and the donner vector by mediation of Cre recombinase (see patent publication No. 3, etc.). It should be noted herein, however, that any technique as reported in the prior art publication does not improve a probability of homologous recombination to such an extent that it is not substantially different from that of any conventional technique which introduces a foreign DNA at a very low probability of homologous recombination.
[Non-patent publication No. 1] Sauer, B., et al., Proc. Natl. Acad. Sci. USA, 85, 5166-5170, 1988
[Non-patent publication No. 2] Gu, H., et al., Cell, 73, 1155-1164, 1993
[Non-patent publication No. 3] Araki, K., Imaizumi, K., Oike, Y and Yamamura, K., J. Biochem. (Tokyo), 122, 977-982, 1997
[Non-patent publication No. 4] Lakso, M., et al., Proc. Natl. Acad. Sci. USA, 89, 6232-6236, 1992
[Non-patent publication No. 5] Rosant, J., et al., Nature Med., 1, 592-594, 1995
[Non-patent publication No. 6] Araki, K., Araki, M., and Yamamura, K., Nucleic Acids Res., 2002, Vol.30, No.19 e103
[Non-patent publication No. 7] Albert, H., et al., Plant J, 7, 649-659, 1995
[Non-patent publication No. 8:] Araki, K., Araki, M. and Yamamura, K.: Nucleic Acid Res. 25, 868-872, 1997
[Patent publication No. 1] WO01/05987A1
[Patent publication No. 2] Japanese Patent Publication No. 2002-345477 A1
[Patent publication No. 3] Japanese Patent Publication No. 2006-333742A1

### DISCLOSURE OF INVENTION

### PROBLEMS TO BE SOLVED BY INVENTION

As a result of extensive research to establish a technique for introducing a mutation of a foreign gene into a target gene at a high probability, it has been found by the present inventors that a target recombinant gene produced by homologous recombination of the target gene into which to introduce the foreign gene DNA is recombined with a target recombinant vector (targeting vector) carrying the foreign gene DNA by mediation of a Cre-mutant lox system using a mutation introduction cassette of a target introduction vector formed so as to contain the mutant gene DNA as an object of introduction, thereby enabling a precise and ready introduction of such the mutation of the foreign gene into a target gene at a high probability. It has also been found that any mutant gene can be introduced regardless of the kind of a target gene by utilizing such a mutation introduction cassette. Moreover, it has been found that the method for the introduction of the target mutant gene can form knock-in non-human mammalian animals, particularly knock-in mice, without great difficulty and for a short period of time. The present invention has been completed on the basis of these findings.

Therefore, the present invention has the object to provide a method for introducing a gene mutation comprising introducing a mutation of a foreign gene into a target gene at a high probability regardless of the kind of a target gene by utilizing a mutation introduction cassette and a Cre-mutant loxP system.

The present invention has also the object to provide the method for introducing the gene mutation, comprising recombining a target DNA sequence carried in the target recombinant gene on a chromosome with a mutation-introduced DNA sequence region contained in the mutation introduction cassette of the mutation introduction vector by mediation of a Cre recombinase.

In a preferred embodiment, the present invention has the object to provide the method for introducing the gene mutation wherein the target recombinant gene as an object of mutation introduction is produced by homologous recombination of the target gene with the target introduction vector.

In a further preferred embodiment, the present invention has the object to provide the method for introducing the gene mutation, wherein the mutation introduction cassette of the mutation introduction vector comprises a mutant DNA sequence and a second positive selection marker DNA sequence flanked by a pair of a third mutant lox sequence and a fourth mutant lox sequence.

In a further preferred embodiment, the present invention has the object to provide the method for introducing the gene mutation, wherein the target DNA sequence flanked by a pair of the first mutant lox sequence and the second mutant lox sequence carried in the target recombinant gene is recombined with the mutant DNA sequence flanked by a pair of the third mutant lox sequence and the fourth mutant lox sequence contained in the mutation introduction cassette of the mutation introduction vector by mediation of the Cre recombinase.

In a still further preferred embodiment, the present invention has the object to provide the method for introducing the gene mutation, wherein each of the mutant lox sequences comprises a mutant lox sequence carrying a mutation in a spacer sequence of the loxP sequence or in 5 -pair inverted repeats or 3 -pair inverted repeats. Such mutant lox sequences may include, for example, lox71, lox2272 or loxKMR3.

In a still further preferred embodiment, the present invention has the object to provide the method for introducing the gene mutation, wherein the mutation-introduced gene obtained by recombination of the target recombinant gene with the mutation introduction cassette of the mutation introduction vector comprises a mutant DNA sequence flanked by a pair of a fifth mutant lox sequence such as lox71/KMR3, etc., and a sixth mutant lox sequence such as lox2272, etc.

In a still further preferred embodiment, the present invention has the object to provide the method for introducing the gene mutation, comprising the recombination step of homologously recombining a first DNA sequence region of the target gene with a second DNA sequence region of the target recombinant vector to form the target recombinant gene by recombining a mutation introduction exon as an object of mutation introduction carried in the first DNA sequence region with a target DNA sequence carried in the second DNA sequence region; and the mutation introduction step of recombining a pair of the first mutant lox sequence and the second mutant lox sequence contained in a third DNA sequence region of the target recombinant gene with a mutation target DNA sequence region flanked by the mutant lox sequence pair via mediation of the Cre-mutant lox system composed of the Cre-recombinase and the mutant lox sequence pair.

In a still further preferred embodiment, the present invention has the object to provide the method for introducing the gene mutation, wherein the mutant DNA sequence contained in each of the mutation introduction cassette and the mutation-introduced gene is a mutant exon.

In a still further preferred embodiment, the present invention has the object to provide the method for introducing the gene mutation, wherein the target gene is a gene derived from subunit KCNQ2 of the voltage-gated potassium channel gene of human autosomal dominant benign familial neonatal convulsion (BFNC), and the target mutation of the target gene is a mutant Y284C (SQ ID NO: 1 and NO: 2) or A306T (SQ ID NO: 3 and NO: 4) of the KCNQ2 subunit gene.

In another aspect, the present invention has the object to provide a method for forming the mutation-introduced gene by introducing the gene mutation into the target gene by the method for introducing the gene mutation as described above.

In another aspect, the present invention has the object to provide the mutation-introduced gene containing the mutant DNA sequence carrying the mutant exon flanked by a pair of the fifth mutant lox sequence and the sixth mutant lox sequence.

In another aspect, the present invention has the object to provide a mutation introduction cassette of a variable type, which comprises the mutant DNA sequence carrying the mutant exon flanked by a pair of the third mutant lox sequence and the fourth mutant lox sequence.

In anther aspect, the present invention has the object to provide the mutation introduction vector containing the mutation introduction cassette.

In another aspect, the present invention has the object to provide a mutation-introduced ES cell, wherein the mutation-introduced gene introduced therein is a mutation-introduced gene derived from the KCNQ2 subunit of the voltage-gated potassium channel gene of human autosomal dominant benign familial neonatal convulsion (BFNC). Such a mutation may include, for example, mutant Y284C or A306T of the KCNQ2 subunit gene.

In another aspect, the present invention has the object to provide a knock-in non-human mammalian animal, in particular a knock-in mouse, wherein the mutation-introduced gene introduced therein is a mutant gene derived, for example, from the KCNQ2 subunit of the voltage-gated potassium channel gene associated with the human autosomal dominant benign familial neonatal convulsion (BFNC), and it comprises mutant Y284C or A306T of the KCNQ2 subunit gene.

### MEANS TO SOLVE THE PROBLEMS

In order to achieve the objects as described above, the present invention provides the method for introducing the gene mutation, which comprises recombining the target DNA sequence contained in the target gene on a chromosome and flanked by a pair of the first mutant lox sequence and the second mutant lox sequence with the mutant DNA sequence carried in the mutation introduction cassette and flanked by a pair of the third mutant lox sequence and the fourth mutant lox sequence via the mutation introduction cassette of the mutation introduction vector by mediation of the Cre recombinase and introducing the recombinant mutant DNA sequence into the gene of interest.

In a preferred embodiment, the present invention provides the method for introducing the gene mutation, wherein the target recombinant gene is a recombinant gene which carries the target DNA sequence flanked by a pair of the first mutant lox sequence and the second mutant lox sequence different therefrom and which is produced by homologously recombining the first DNA sequence region of the target gene with the second DNA sequence region of the target recombinant vector.

In a further preferred embodiment, the present invention provides the method for introducing the gene mutation, wherein the mutation introduction cassette of the mutation introduction vector includes the mutant DNA sequence and a second positive selection marker DNA sequence, both being flanked by a pair of the third mutant lox sequence and the fourth mutant lox sequence.

In a still further preferred embodiment, the present invention provides the method for introducing the gene mutation, wherein the mutant DNA sequence carried in the mutation introduction cassette of the mutation introduction vector and flanked by a pair of the third mutant lox sequence (52) and the fourth mutant lox sequence (56) is recombined with the target DNA sequence contained in the target recombinant gene and flanked by a pair of the first mutant lox sequence and the second mutant lox sequence by mediation of the Cre recombinase.

In a still further preferred embodiment, the present invention provides the method for introducing the gene mutation, wherein the first mutant lox sequence comprises a mutant lox sequence carrying a mutation in its 5 -terminal inverted repeat of the loxP sequence, such as lox71; the second mutant lox sequence comprises a mutant lox sequence carrying a mutation in the spacer sequence of the loxP sequence, such as lox2272; the third mutant lox sequence comprises a mutant lox sequence carrying a mutation in the 3-terminal inverted repeat of the loxP sequence, such as loxKMR3; and the fourth mutant lox sequence comprises a mutant lox sequence carrying a mutation in the spacer sequence of the loxP sequence, such as lox2272.

In a still further preferred embodiment, the present invention provides the method for introducing the gene mutation, wherein the mutation-introduced gene formed by recombination of the target recombinant gene with the mutation introduction cassette of the mutation introduction vector comprises a mutation-introduced DNA sequence region which carries the mutant DNA sequence and a second positive selection marker DNA sequence and which is flanked by a pair the fifth mutant lox sequence and the sixth mutant lox sequence, the fifth mutant lox sequence being formed by recombination of the first mutant lox sequence with the third mutant lox sequence, and the sixth mutant lox sequence being formed by recombination of the second mutant lox sequence and the fourth mutant lox sequence, the fifth mutant lox sequence including, e.g., lox71/KMR3, and the sixth mutant lox sequence including, e.g., lox2272.

In a still further preferred embodiment, the present invention provides the gene mutation introduction method for introducing a foreign mutant gene into the target gene, comprising the homologous recombination step for forming a target recombinant gene (40) by homologously recombining a target gene (10) with a target recombinant vector (20) to thereby recombine a mutation introduction exon (12) as an object of mutation introduction carried in a first DNA sequence region (10a) of the target gene (10) with a target DNA sequence (32) contained in a second DNA sequence region (20a) of the target recombinant vector (20); and the mutation introduction step for forming a mutation-introduced gene (60) with a mutation-introduced DNA sequence region (54) introduced therein by (specifically) recombining a mutation target DNA sequence region (35), which is carried in a third DNA sequence region (40a) of the target recombinant gene (40) and flanked by a pair of the first mutant lox sequence (30) and the second mutant lox sequence (36), with a mutation target DNA sequence region (35) flanked by the pair thereof via the mutation introduction cassette (51) of the mutation introduction vector (50) by mediation of a Cre-mutant lox system composed of the Cre recombinase and a pair of the mutant lox sequences to form a mutation-introduced gene (60);

wherein the target recombinant vector (20) comprises a negative selection marker DNA sequence (22), a first homologous recombination DNA sequence region (24), a target DNA sequence region (26), and a second homologous recombination DNA sequence region (34), and the target DNA sequence region (26) comprises the first mutant lox sequence (30), a target DNA sequence (32), a first positive selection marker DNA sequence (34), the second mutant lox sequence (36) and a polyA addition sequence (38), and the first and second homologous recombination DNA sequence regions (22, 28) have each a length of the DNA sequence required for homologous recombination;

wherein the third DNA sequence region (40a) of the target recombinant gene (40) comprises the first homologous recombination DNA sequence region (24), the first mutant lox sequence (30), the mutation target DNA sequence region (35), the second mutant lox sequence (36) and the second homologous recombination DNA sequence region (28), and the mutation target DNA sequence region (35) comprises the target DNA sequence (32) and the first positive selection marker DNA sequence (34);

wherein the mutation introduction cassette (51) of the mutation introduction vector (50) comprises the third mutant lox sequence (52), the mutation-introduced DNA sequence region (54) and the second mutant lox sequence (56), and the mutation-introduced DNA sequence region (54) comprises a mutant DNA sequence (57) and a second positive selection marker DNA sequence (58); and

wherein the mutation-introduced gene (60) comprises the first homologous recombination DNA sequence region (24), the fifth mutant lox sequence (62), the mutation-introduced DNA sequence region (54), the sixth mutant lox sequence (66) and the second homologous recombination DNA sequence region (28), and the mutation-introduced DNA sequence region (54) comprises the mutant DNA sequence (57) and the second positive selection marker DNA sequence (58).

In a still further preferred embodiment, the present invention provides the method for introducing the gene mutation, wherein the mutant DNA sequence of the mutation introduction cassette of the mutation introduction, vector or the mutation-introduced gene comprises a mutant exon.

In a still further preferred embodiment, the present invention provides the method for introducing the gene mutation, wherein mutant Y284C (SQ ID NO: 1 and NO: 2) or A306T (SQ ID NO: 3 and NO: 4) derived from the KCNQ2 subunit of the voltage-gated potassium channel gene of the human autosomal dominant benign familial neonatal convulsion (BFNC) is introduced as the mutant gene.

In another aspect, the present invention provides a method for forming the mutation-introduced gene wherein the mutation-introduced gene is formed by introducing a gene mutation into the target gene by the method for introducing the gene mutation as described above.

In another aspect, the present invention provides a mutation-introduced gene, wherein the mutation-introduced gene comprises the first homologous recombination DNA sequence region, the fifth mutant lox sequence, the mutation-introduced DNA sequence region, the sixth mutant lox sequence and the second homologous recombination DNA sequence region, and the mutation-introduced DNA sequence region comprises the mutant DNA sequence and the second positive selection marker DNA sequence and it is flanked by a pair the fifth mutant lox sequence and the sixth mutant lox sequence.

In a preferred embodiment, the present invention provides the mutation-introduced gene, wherein a pair of the fifth mutant lox sequence and the sixth mutant lox sequence is formed by recombination of a pair of the first and second mutant lox sequences with a pair of the third and fourth mutant lox sequences.

In another aspect, the present invention provides a mutation introduction cassette of a variable type comprising a pair of the third mutant lox sequence, e.g., loxKMR3, and the fourth mutant lox sequence, e.g., lox2272, as well as the mutant DNA sequence and the DNA sequence of the second positive selection marker, flanked by the pair thereof.

In another aspect, the present invention provides the mutation introduction vector carrying the mutation introduction cassette as described above.

In a further aspect, the present invention provides a knock-in non-human mammalian animal, particularly mouse, into which the mutation-introduced gene as described above has been introduced. In a preferred embodiment, the present invention provides the knock-in non-human animals such as knock-in mice, carrying a gene mutant Y284C or A30GT derived from the KCNQ2 subunit of the voltage-gated potassium channel gene associated with the human benign familial neonatal convulsion (BFNC).

### BRIEF DESCRIPTION OF THE ACOMPANYING DRAWINGS

[Fig. 1] A schematic illustration of sequence configurations of the target gene and the target recombinant vector (20) containing the target DNA sequence (32).
[Fig. 2] A schematic inllustration of a sequence configuration of the target recombinant gene formed by homologous recombination of the target gene with the target recombinant vector.
[Fig. 3] A schematic illustration of a sequence configuration of the mutation introduction cassette contained in the mutation introduction vector and a sequence configuration of the mutation-introduced gene with the gene mutation introduced therein by recombination of the target recombinant gene with the mutation introduction cassette by mediation of the Cre-mutant lox sequence system.
[Fig. 4] A schematic illustration of a sequence configuration of the target gene derived from the KCNQ2 subunit of the voltage-gated potassium channel gene associated with the human autosomal dominal benign familial neonatal convulsion (BFNC) and a sequence configuration of a targeting vector for homologous recombination.
[Fig. 5] A schematic illustration of sequence configurations of the target gene KCNQ2 and the targeting vector as well as a sequence configuration of the mutation introduction vector containing mutation Y284C or A306T as the mutant gene.
[Fig. 6] A schematic illustration of a sequence configuration of the mutation introduction vector containing the mutation Y284C and A306T of the KCNQ2 gene as the mutant DNA sequence.
[Fig. 7] A schematic illustration showing procesures for forming a mouse ES cell with a DNA sequence portion of the KCNQ2 gene by introducing the target recombinant vector into the mouse ES cells.
[Fig. 8] A schematic illustration showing procedures for forming the mouse mutant ES cells with the mutant DNA sequence portion of the KCNQ2 gene introduced therein by homologous recombination of the mouse ES cells prepared by the procedures as shown in Fig. 7 with the mutation introduction vector.
[Fig. 9] A schematic illustration showing procedures for forming a mutant knock-in mouse from the mouse mutant ES cells formed by the procedures as shown in Fig. 8.

### MODES FOR CARRYING OUT THE INVENTION

The present invention relates to a method for introducing a gene mutation for introducing a gene mutation into a target gene. The method for introducing the gene mutation is characterized by introducing a mutant DNA sequence corresponding to a foreign mutant gene into a mutation introduction exon (i.e., a target DNA sequence) having genetic functions of the target gene by two stages.

The first stage of the method for introducing the gene mutation of the present invention is involved in the homologous recombination step in which the DNA sequence of the mutation introduction exon as an object for introduction of the gene mutation of the target gene (a target DNA sequence) is recombined to allow an introduction of the gene mutation of interest to form the target recombinant gene. The second stage is involved in the recombination (specific recombination) step in which the target DNA sequence of the target recombinant gene formed in the first stage is replaced into the mutant DNA sequence of interest using the mutation introduction vector (i.e., a targeting vector).

In order to allow an efficient recombination of the target DNA sequence with the foreign mutant DNA sequence of interest, the homologous recombination step of the method for introducing the gene mutation according to the present invention may be constructed by recombining the target gene on a chromosome with the target DNA sequence portion flanked by a pair of the first mutant lox sequence and the second mutant lox sequence different from the first mutant lox sequence. To this recombination modification method may be used any conventional methods known to the art, and it may be constructed generally by recombination of the DNA sequence region carrying the target DNA sequence of the target gene by homologous recombination of the target gene with the target recombinant vector (i.e., the targeting vector).

It is now to be noted herein that the recombination step of the first stage for the gene mutation introduction method according to the present invention may recombine and alter the target gene by the homologous recombination process, that is, a gene targeting process, using the target recombinant vector called a targeting vector. This homologous recombination step may be carried out by introducing the target recombinant vector carrying the target DNA sequence as a target of mutation introduction, i.e., the targeting vector, by means of conventional vector introduction techniques such as electroporation, etc. into mouse ES cells and so on. The homologous recombination method has the drawback, however, that a probability of recombination is very low, i.e., as low as 0.01% or less.

Furthermore, in order to improve the defects of this homologous recombination method, the present invention enables an introduction of the gene mutation of interest into the mutation-introduced gene at a very high probability and for a very short period of time by constructing the specific recombination step of the second stage thereof so as to have such a sequence configuration as will be described hereunder.

The recombination step of the second stage for the gene mutation introduction method for introducing the gene mutation according to the present invention may be involved in recombining the target recombinant gene obtained by recombation and alteration in the manner as described above using the mutation introduction vector and specifically recombining the target DNA sequence of the target recombinant gene with the mutant DNA sequence carried in the mutation introduction vector. This recombination step may recombine the mutant DNA sequence carried in the mutation introduction vector with the target DNA sequence of the target recombinant gene at a very high probability as high as approximately 100%. In other words, the present invention has the great characteristics that the mutation introduction vector, in particular the mutation introduction cassette, is devised and constructed so as to introduce the target DNA sequence into the target recombinant gene and thus the mutant DNA sequence of interest therein at a very high probability and with rapidity.

By carrying out the method for introducing the gene mutation according to the present invention at two stages in the manner as described above, the present invention has the great features that it can be applied to any gene regardless of the kinds of genes. In the present invention, once the target recombinant gene is produced by recombining and altering the mutation introduction exon (i.e., the target DNA sequence) as an object of mutation introduction via the target recombinant vector at the first stage, the gene mutation of interest can be introduced into the mutation introduction exon of the target recombinant gene by the mutation introduction vector at the second stage at an extreme high probability and with rapidity, the mutation introduction vector carrying the mutation introduction cassette containing the gene mutation of interest. In other words, although the present invention is carried out at the first stage of the method to produce the target recombinant gene by conventional homologous recombination so that a probability of recombination of the target recombinant gene is very low, the gene mutation can be recombined and introduced at an extremely high probability and with rapidity at the second stage of the method using the mutation introduction vector carrying the mutation introduction cassette with the gene mutation of interest introduced therein, once the target recombinant gene has been formed at the first stage thereof. Therefore, even if the gene mutation of interest be changed for another one, the mutation introduction cassette carrying another gene mutation can be produced for a very short period of time so that the another gene mutation of interest can be introduced into a target gene with rapidity at a high probability and for a very short period of time. Furthermore, as the mutation introduction cassette carrying the gene mutation of interest and the mutation introduction vector carrying the mutation introduction cassette can be formed rapidly and within short in conventional manner so that, eventually, knock-in mammalian animals having another gene mutation replaced by the original one can be produced rapidly at an extremely high probability and for a very short period of total time.

Therefore, as described above, the present invention have the extreme great advantages that it can be applied in substantially the same manner to any gene, regardless of the kinds of genes, even if the target gene would be any disease-associated gene including an epilepsy-associated gene or a tumor-associated gene, or any other gene and it can produce the mutation-introduced gene carrying the gene mutation of interest introduced therein at a high probability and with rapidity. Further, likewise, the present invention has the great advantage that a mutant DNA sequence of interest can be introduced into any DNA sequence having functions as any target gene, i.e., an exon DNA sequence.

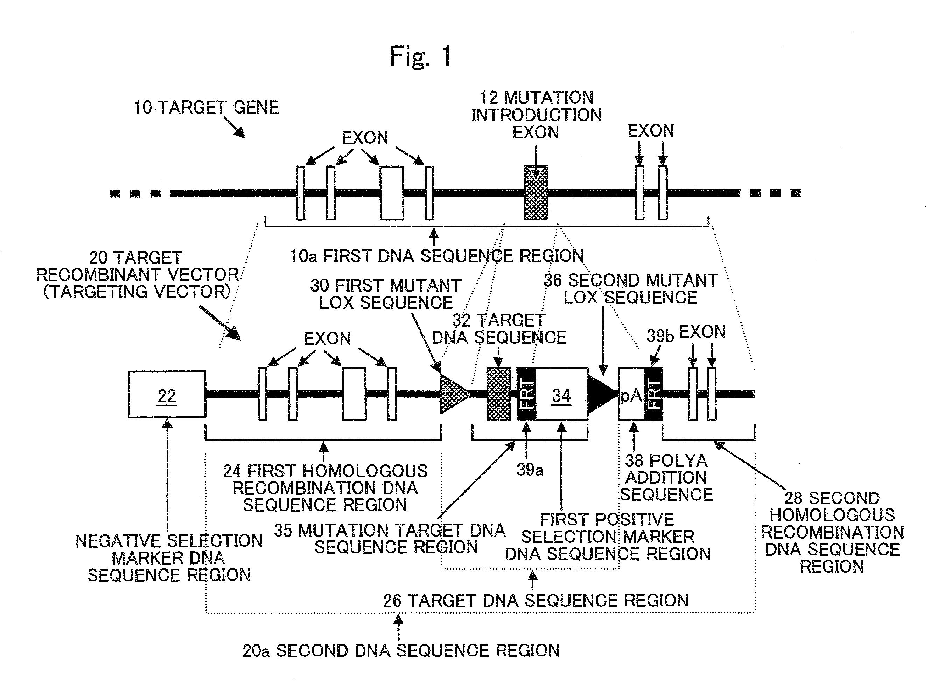

A detailed description will be made regarding the present invention with reference to Figs. 1 to 6. It should be noted herein, however, that the present invention should not be understood to be limited to any modes and embodiments as represented in the drawings attached hereto and the accompanying drawings in turn are not construed as limiting the invention in any respect to those modes and embodiments of the accompanying drawings. Further, any improvements and modifications departing from the modes and embodiments represented in the accmpanying drawings should be understood as being encompassed within the scope of the present invention.

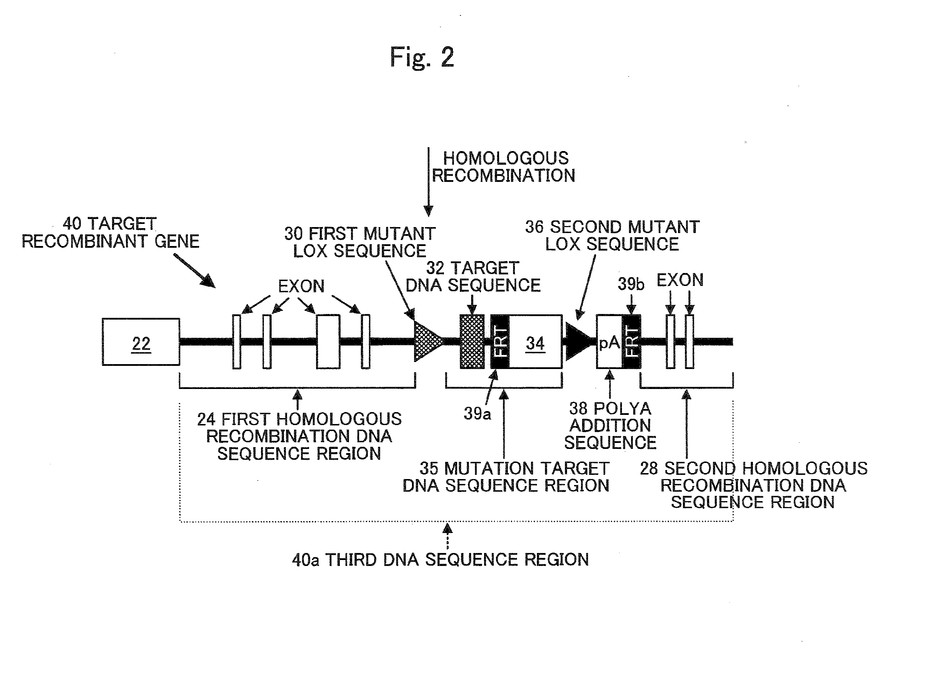

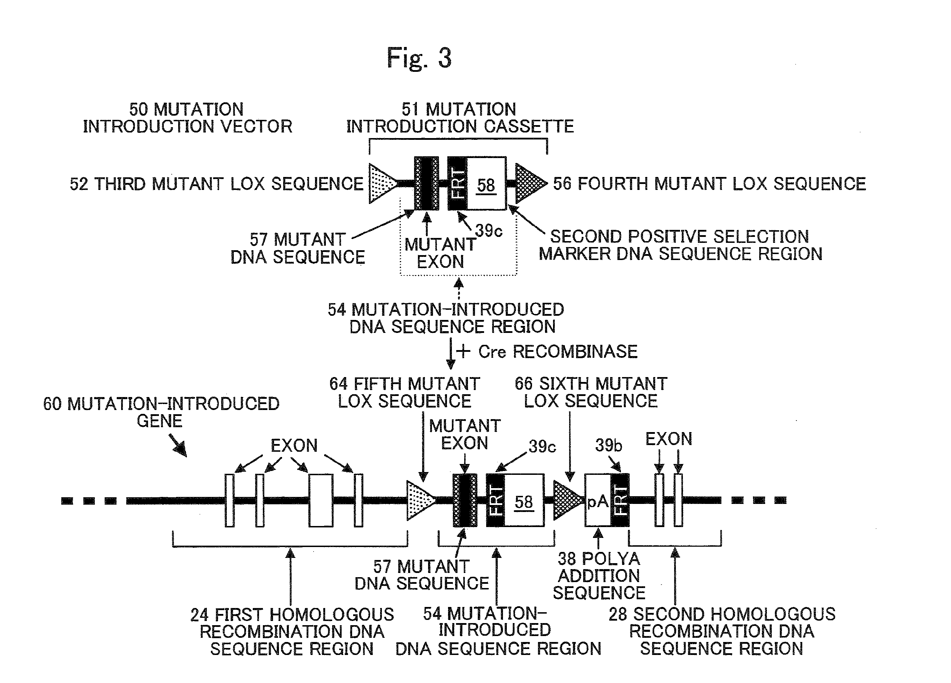

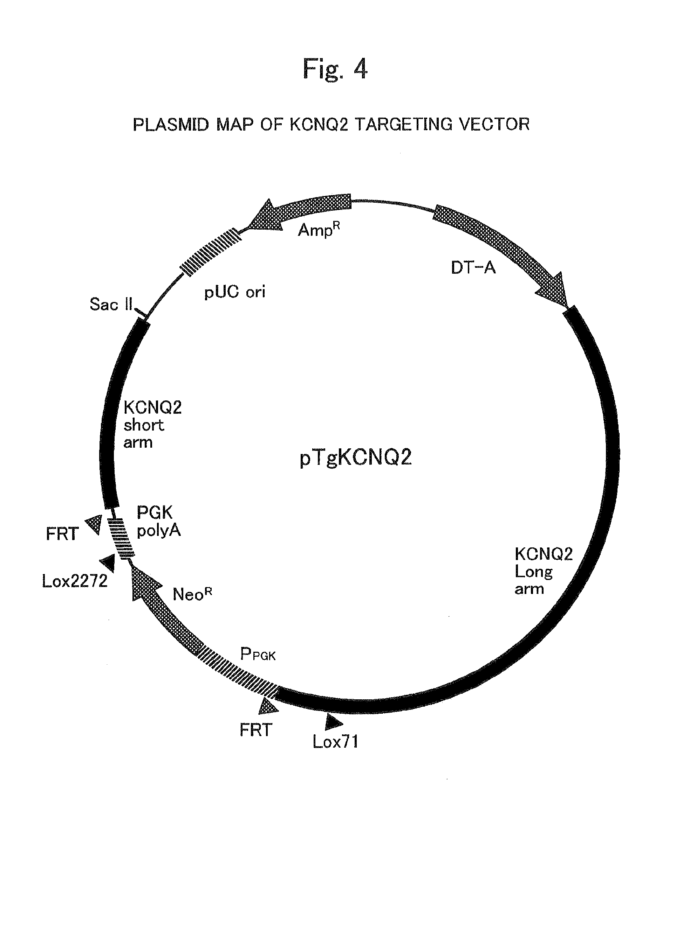

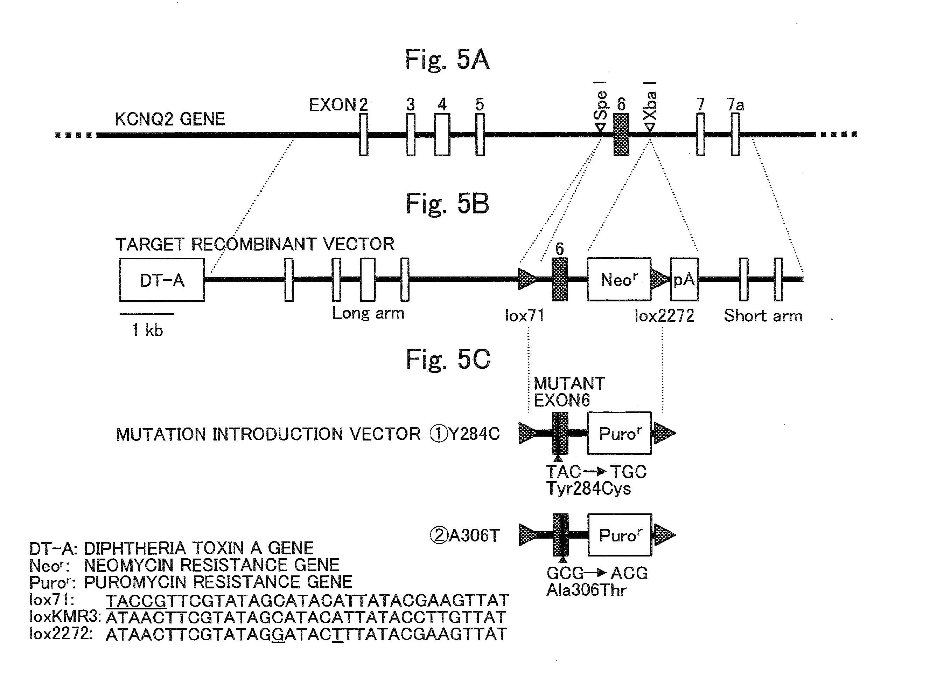

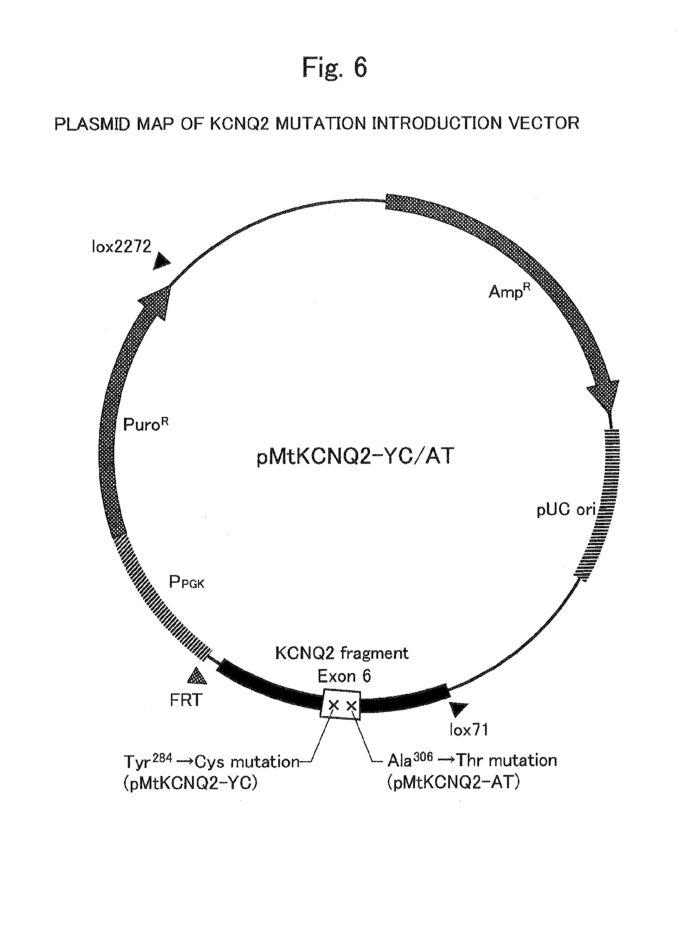

Fig. 1 is a schematic illustration showing a sequence configuration of the target gene (10) into which the gene mutation is to be introduced, and a sequence configuration of the target recombinant vector (20) carrying the target DNA sequence (32) to be recombined with the target gene (10) by homologous recombination. Fig. 2 is a schematic illustration showing a sequence configuration of the target recombinant gene (40) obtained by the homologous recombination of the target gene (10) with the the target recombinant vector (20). Fig. 3 is a schematic illustration showing a sequence configuration of the mutation introduction cassette (51) of the mutation introduction vector (50), and a sequence configuration of the mutation-introduced gene (60) carrying the gene mutation introduced therein by recombination of the target recombinant gene (40) with the mutation introduction cassette (51) by mediation of the Cre-mutant lox sequence system. Fig. 4 is a schematic illustration showing a sequence configuration of the target gene derived from the KCNQ2 subunit of the voltage-gated potassium channel gene associated with the human autosomal dominant benign familial neonatal convulsion (BFNC) for use with homologous recombination. Fig. 5 is a schematic illustration showing a sequence configuration of the target gene KCNQ2 and the targeting vector as well as a sequence configuration of the mutation introduction vector carrying mutation Y284C or A306T as a mutant gene. Fig. 6 is a schematic illustration showing a sequence configuration of the mutation introduction vector carrying the mutation Y284C or A306T. The schematic illustration as indicated in each of the above figures is shown to be illustrative solely for brevity of explanation of the invention, and the present invention is also to be understood as failing to be limited in any respect to the attached drawings.

First, the target gene (10) to be used for the method for introducing the gene mutation according to the present invention has the great merits and advantages that it can be applied to any gene without limitation to particular kinds of genes as described above. In this description, for brevity of explanation, the description will be made by taking the gene derived from the KCNQ2 subunit of the voltage-gated potassium ion channel gene associated with the human autosomal dominant benign familial neonatal convulsion (BFNC) as an epilepsy-associated gene as shown in Fig. 5 as an example of the target gene (10) as shown in Fig. 1, and the mutation Y284C or A306T as shown in Fig. 6 as the mutant gene as an object of introduction of gene mutation.

In the gene mutation introduction method according to the present invention, the target gene (10) as a target of introduction of gene mutation may be selected from any gene, which carries a mutation introduction exon (12) that is expected to be provided with functions of interest as a gene. In selecting the target gene, i.e., the mutation introduction exon (target DNA sequence), as an object of introduction for gene mutation, it is advantageous, as a matter of course, to select it from any gene in which the objective gene mutation has already been known or from a known gene mutation. In accordance with the present invention, more specifically, it is preferred to select a gene derived from the KCNQ2 subunit of the voltage-gated potassium channel gene associated with the human autosomal dominant benign familial neonatal convulsion (BFNC) which is known to be an epilepsy-associated gene, and to possess a known gene mutation and to select exon 6 carrying the known mutation Y284C (SQ ID NO: 1 and NO:2) or A306T (SQ ID NO:3 and NO:4) as the target DNA sequence. It is further to be noted herein that any other exon may be selected from a genomic gene with freedom as far as it can meet with the object for introduction of gene mutation.

The target gene (10) as selected in the manner as described above may be recombined and modified with the target recombinant vector (targeting vector) (20) by the homologous recombination method known to the art, i.e., gene targeting method. The homologous recombination method may be carried out in conventional manner by introducing the target recombinant vector, i.e., the targeting vector, carrying the target DNA sequence as a target for mutation introduction into mouse ES cells and so on by conventional vector introduction techniques known in the art, such as electroporation, etc. The homologous recombination method is the technology which has already been established in the art so that detailed descriptions will be omitted herefrom.

The target recombinant vector (20) to be used herein may be a vector that allows an introduction of mutant lox sequences in order to facilitate the mutation introduction into a specific target gene present in the genomic DNA of ES cells such as mouse ES cells and so on. It may be formed by introducing it into a plasmid as a base by conventional techniques known to the art in order to introduce the mutation of interest into the target gene. As such plasmids, there may be used any plasmid which is used for conventional techniques known to the art without limitation to any particular one, and they may include, for example, pBluescript II SK+, pGEM, pBR322, pUCs such as pUC18, pUC19, pUC118, pUC119, etc., pSPs such as pSP64, pSP65, pSP73, etc., and pGEMs such as pGEM-3, pGEM-4, pGEM-3Z, etc. It may also be selected appropriately for subsequent insertion to construct a target introduction vector by taking into consideration kinds of cleavage sites and the order of sequences present in DNA fragments to be cleaved by restriction enzymes.

A genomic DNA fragment to be used for the construction of the target recombinant vector for the present invention may be produced, for example, by obtaining information on the sequence of DNA bases of the target gene including without limitation to the KCNQ2 gene, etc., and amplifying the necessary region of the gene based on this information by PCR using a mouse clone of bacterial artificial chromosome (BAC), etc., as a template, or using a clone isolated from the genome library. Among the base sequence fragments of the KCNQ2 gene obtained by amplifying in the manner as described above, the exon portion being the target site of mutation introduction may be preferably designed, for example, to carry a first homologous recombination DNA sequence region of approximately 5.5 kb and a second homologous recombination DNA sequence region of approximately 2 kb. Each oligonucleotide of the oligonucleotide pair may also be prepared by conventional methods used in the art. Further, the first and second homologous recombination DNA sequence regions may be produced by producing each of them separately and then combining them together into one.

In addition, the terms "target recombinant vector" used herein are intended to mean a targeting vector which acts as a vector of the target DNA sequence to be introduced into the target gene from the outside. The target recombinant vector to be used for the present invention may be formed in conventional manner known to the art.

The target recombinant vector (20) may be generally constructed so as to make the length of the DNA sequence of the first homologous recombination DNA sequence region (24) longer than that of the DNA sequence of the second homologous recombination DNA sequence region (28). In this description, the first homologous recombination DNA sequence region (24) will be described hereinafter as a "long-arm region" or its related term, and the second homologous recombination DNA sequence region (28) will be described hereinafter as a "short-arm region" or its related term, for brevity of explanation. It is to be noted herein, however, that they are not bound to those terms. In other words, the DNA sequence length of the first homologous recombination DNA sequence region (24) may be shorter than or equal to that of the second homologous recombination DNA sequence region (28).

In order to effectively perform the mutation introduction by recombination, the target recombinant vector (20) may be constructed in such a manner as will be described hereinafter. More specifically, the target recombinant vector (20) may be constructed, for example, so as to possess the second DNA sequence region (20a) having the DNA sequence substantially identical to the first DNA sequence region (10a) carrying the mutation introduction exon (12) of the target gene (10) and to flank the target DNA sequence (32) by a pair of the first mutant lox sequence (30) and the second mutant lox sequence (36) different therefrom.

It is to be noted that the terms "substantially identical to" are used herein to mean the case where both are thoroughly identical to each other and also the case where both maintain substantially the same identities to the extent that their original properties and features and the like are not impaired.

Here, the terms "mutant lox sequence" will be described more in detail. The terms "mutant lox sequence" as used herein are intended to mean a mutant lox sequence in which one base or more bases is or are replaced by another base or bases in the spacer sequence located in the center of the loxP sequence and composed of 8 bases and/or the 5 -pair inverted repeats and/or the 3 -pair inverted repeats located on both respective sides thereof and composed of 13 bases each.

More specifically, the terms "first mutant lox sequence" used herein are intended to mean a mutant lox sequence in which a mutation is located in the 5 -pair inverted repeats of the loxP sequence on the 5 -terminal side of the target DNA sequence. The first mutant lox sequence may include lox71. The terms "second mutant lox sequence" used herein are intended to mean a mutant lox sequence in which a mutation is located in the spacer sequence of the loxP sequence on the 3 -terminal side of the target DNA sequence. The second mutant lox sequence may include lox2272. The lox71 is a mutant lox sequence in which the first five bases on the 5 -terminal side of 13 bases (ATAACTTCGTATA) of the 5 -pair inverted repeats are mutated to TATTG. On the other hand, the lox2272 is a mutant lox sequence in which the spacer sequence of the loxP sequence is mutated and the second base C of 8 bases (GCATACAT) is mutated to base G and the sixth base A thereof is mutated to base T.

Now, a detailed description will be made hereinafter regarding a sequence configuration of the target recombinant vector (20) with reference to Fig. 1. The target recombinant vector (20) possesses the second DNA sequence region (20a) carrying the DNA sequence substantially identical to the second DNA sequence region (10a) of the target gene (10). The second DNA sequence region (20a) may be composed of the first homologous recombination DNA sequence region (24), the first mutant lox sequence (30), the target DNA sequence region (26), the second mutant lox sequence (36), a polyA addition sequence (38) and the second homologous recombination DNA sequence region (28). The target DNA sequence region (26) flanked by a pair of the first mutant lox sequence (30) and the second mutant lox sequence (36) may be composed of the target DNA sequence (32) and the DNA sequence of the first positive selection marker (34). Further, FRT sequences (39a, 39b) may be located between the target DNA sequence (32) and the first positive selection marker DNA sequence (34) on the side of the polyA addition sequence (38). The DNA sequences of the first homologous recombination DNA sequence region (24) and the second homologous recombination DNA sequence region (28) are generally as long as approximately 5 kb and 2 - 3 kb, respectively. The first homologous recombination DNA sequence region (24) and the second homologous recombination DNA sequence region (28), respectively, may carry exon DNA sequences and intron DNA sequences.

Furthermore, the target recombinant vector (20) may be provided with a negative selection marker DNA sequence (22) on the 5 -terminal side of the first homologous recombination DNA sequence region (24). As the negative selection marker (22), there may be used, for example, a gene capable of producing a cytotoxic protein such as a diphtheria toxin A fragment gene (DT-A), etc. or a gene capable of altering metabolisms of toxic substances such as herpes virus thymidine kinase gene (HSV-tk), etc. On the other hand, the first positive selection marker DNA sequence (34) incorporated in the target DNA sequence region (26) of the target recombinant vector (20) may include, for example, a drug resistance gene marker such as neomycin resistance gene (Neo^{R}). By locating such a drug resistance gene marker in the manner as described above, only the cells in which the homologous recombination has occurred with the target site of the genomic DNA in the vector are selected upon incorporation of the vector into the cells.

The target recombinant vector having the above sequence configuration may be produced, for example, by cleaving a pBluescript II SK+ plasmid with an appropriate restriction enzyme, incorporating DT-A into the cleaved site and cleaving the resulting plasmid with an appropriate restriction enzyme, followed by incorporating a long-arm - short-arm fragment into the cleaved site, cleaving the resulting long-arm-short-arm fragment with an appropriate restriction enzyme, incorporating a lox71 sequence fragment into the cleaved site, cleaving a portion upstream by appropriately 200 bp from the terminus of the particular exon of the long-arm region, incorporating a drug resistance gene marker, etc. into the resuting cleaved site, cleaving the particular portion carrying pBluescript II SK+, and linearizing the sequence. The resulting target recombinant vector can be confirmed by determination of its base sequence.

The target recombinant vector (20) having the above sequence configuration may then be introduced into mouse ES cells and so on by conventional vector introduction techniques such as electroporation, etc. This may allow a homologous recombination of the target gene (10) with the target recombinant vector (20) on a chromosome resulting in the formation of the target recombinant gene (40) carrying the target DNA sequence (32) as a target for mutation introduction. As a result of the homologous recombination, the resulting target recombinant gene (40) is provided with a third DNA sequence region (40a) having a sequence configuration substantially identical to that of the second DNA sequence region (20a) of the target recombinant vector (20) (see Fig. 2).

As shown in Fig. 2, the third DNA sequence region (40a) of the target recombinant gene (40) has a sequence configuration composed of a negative selection marker DNA sequence (22), the first homologous recombination DNA sequence (24), the first mutant lox sequence (30), the mutation target DNA sequence (35), the second mutant lox sequence (36), a polyA addition sequence (38), a FRT sequence (39b), and the second homologous recombination DNA sequence (28). Further, the mutation target DNA sequence (35) has a sequence configuration consisting of the target DNA sequence (32), the FRT sequence (39a) and the first positive selection marker DNA sequence (34).

Then, the target recombinant gene (40) on the chromosome may be recombined with the mutation introduction vector (50) carrying the mutation introduction cassette (51) to give the mutation-introduced gene (60) by mediation of the Cre recombinase - mutant lox system. In this recombination, the mutation target DNA sequence region (35) carried in the third DNA sequence region (40a) of the target recombinant gene (40) is recombined with the mutation-introduced DNA sequence region (54) carried in the mutation introduction cassette (51) of the mutation introduction vector (50), thereby introducing the gene mutation of interest into the target gene and forming the mutation-introduced gene (60).

The mutation introduction cassette (51) to be used for the present invention is a tool that can introduce a mutant DNA sequence (57) into the target gene and that can be applied to any mutation gene, whichever a gene carries a mutation including without limitation to any particular mutant gene. Further, the mutation introduction cassette has the great feature in that it is of a variable type tht can alter a mutant DNA sequence to be introduced into the target gene with freedom. It should be noted herein that the mutation introduction cassette can be formed with comparably easiness. Thus, once a mutation introduction cassette of a variable type is formed for any mutation, the mutation introduction cassette has the great advantage that it can produce the mutation-introduced gene and non-human animals such as mice and so on, in which the mutation-introduced gene is to be incorporated, with readiness at a high probabilty for a short period of time, whichever poorer a probability of recombination of the mutation-introduced gene is.

As shown in Fig. 3, the mutation introduction cassette (51) of such a variable type as described above may be constructed in such a fashion that, in order to allow an efficient recombination with the target DNA sequence (32) of the target recombinant gene (40), it has a structure consisting of the mutant DNA sequence (57) and the second selection marker DNA sequence (58) located on the 3 -terminus of the mutant DNA sequence (57), both being flanked with a pair of the third mutant lox sequence (52) and the fourth mutant lox sequence (56) different therefrom. Further, a FRT sequence (39c) may be preferably located between the third mutant lox sequence (52) and the second positive selection marker DNA sequence (58). The mutation introduction cassette (51) having such a sequence configuration as described above may be produced by conventional techniques known to the art.

It is to be noted herein that the mutant lox sequence as expressed by the terms "third mutant lox sequence" used herein are intended to be used as meaning a mutant lox sequence carrying a mutation located in the 3 -pair inverted repeats of the loxP sequence on the 5 -terminal side of the mutant DNA sequence. As the third mutant lox sequence, there may be mentioned, for example, loxKMR3. The mutant lox sequence as expressed by the terms "fourth mutant lox sequence" used herein is intended to mean a mutant lox sequence, likewise the second mutant lox sequence, which carries a mutation in the spacer sequence and is located on the 3 -terminal side of the mutant DNA sequence. As the fourth mutant lox sequence, there may be mentioned, for example, lox2722.

The loxKR3 as the third mutant lox sequence (52) is a mutant lox sequence having the 3 -pair inverted repeats of the loxP sequence in which a three-base sequence (5 -GAA) located at the fifth to sixth bases from the 3 -terminus thereof is replaced by a three-base sequence (5 -CGG). On the other hand, the lox2722 is a mutant lox sequence carrying a mutation in the spacer sequence of the loxP sequence, likewise the second mutant lox sequence, in which the second base C of the 8-base sequence (GCATACAT) of the spacer sequence is mutated to base G and the sixth base A thereof is mutated to base T.

Further, the second positive selection marker DNA sequence (58) carried in the mutation introduction cassette (51) may be designed so as to include, for example, a drug resistance gene such as puromycin resistance gene, etc. This drug resistance gene allows a selection of ES cells which are provided with a desired mutant DNA sequence. The positive selection marker DNA sequence may be formed, for example, by amplifying fragments having each a specific length using a specific plasmid as a template by means of PCR using a pair of appropriate oligonucleotides as a template, and cleaving a restriction enzyme recognition site artificially added to the 5 -terminus and the 3 -terminus. By locating such a drug selection marker, the vector can select only cells in which a homologous recombination with the target site of the genomic DNA has occurred into cells upon selection of the vector.

In accordance with the present invention, the mutant DNA sequence (57) of the mutation introduction cassette (51) may be constructed so as to consist of a DNA sequence carrying a mutant exon. As the mutant exon, there may be used, for example, a mutant portion, including without limitation to mutation Y284C and A306T, located in the exon 6 portion of the subunit KCNQ2 of the voltage-gated potassium channel gene of the human autosomal dominant benign familial neonatal convulsion (BFNC). In the event where another gene mutation located in the KCNQ2 gene is targeted, the mutation introduction cassette may be construted so as to include such a mutation corresponding to a cDNA fragment carrying all the exons including, for example, exon 6 located downstream, excpt introns. Likewise, this can be applied to all other mutant genes.

The base sequence of the mutation Y284C of the KCNQ2 gene subunit is a mutant base sequence (SQ ID NO:1) in which base A at the 23rd base of exon 6 of the gene KCNQ2 is replaced by base G. In other words, its amino acid sequence is a mutant amino acid sequence (SQ ID NO:2) in which amino acid residue tyrosine at the eighth amino acid of exon 6 is replaced by cysteine. On the other hand, the base sequence of the mutation A306T thereof is a mutant base sequence (SQ ID NO:3) in which base G at the 100th base of exon 6 thereof is replaced by base A, or its amino acid sequence is a mutant amino acid sequence (SQ ID NO:4) in which amino acid residue alanine at the 34th amino acid thereof is replaced by threonine.

The mutant DNA sequence carrying the gene mutation (such as mutation Y284C or A306T) to be used for the present invention is a gene mutation, for example, in which the mutation Y284C or A306T is introduced into the DNA fragment containing exon 6 of mouse gene KCNQ2 and the loxKR3 sequence is added to its 5 -terminus.

For instance, fragments of the gene KCNQ2 carrying the mutation Y284C of exon 6 of mouse gene KCNQ2 may be produced by amplifying the 5 -fragment and the 3 -fragment each carrying the base mutation Y284C by PCR using a pair of oligonucleotides for amplification of an exon 6 edge region/introduction of loxKMR3 sequence and for introduction of mutation Y284C and using the exon 6 edge region of the gene KCNQ2 as a template. These two fragments may be produced so as to have a desired base length by connection and amplification by litigation-independent cloning method and PCR using a pair of oligonucleotides for amplification of the exon 6 edge region/introduction of loxKMR3 sequence.

The fragments of the gene KCNQ2 carrying the mutation A306T may also be produced in substantially the same manner as those carrying the mutation Y284C. Further, exons carrying other mutations may be produced by substantially the same techniques as described above.

The mutation introduction cassette (51) having the above sequence configuration may be introduced into the mutation introduction vector (50) by conventional techniques well known to the art. As the mutation introduction vector, there may be used, for example, vectors which are used as target recombinant vectors, including without limitation to plasmid vectors, such as pBR322, pUCs (pUC18, pUC19, pUC118, pUC119, etc.), Bluescript II, pSPs (pSP64, pSP65, etc.), pGEMs (pGEM-3, pGEM-4, pGEM-3Z, etc.), and so on. The fragments of the gene KCNQ2 carrying the above mutation may be incorporated into an appropriate plasmid, for example, by cleaving the plasmid with a restriction enzyme, introducing the second positive selection marker DNA sequence region such as puromycin resistance gene, etc., into the cleaved site, then cleaving the resulting DNA sequence with an appropriate restriction enzyme, and then incorporating the fragment into the cleaved site to form the mutation introduction vector having the above sequence configuration.

The mutation introduction vector (50) into which the mutation introduction cassette (51) has been introduced is then recombined to form the mutation-introduced gene (60), for example, by recombination with the target recombinant gene (40) by mediation of the Cre recombinase, thereby incorporating a pair of the third mutant lox sequence (52) and the fourth mutant lox sequence (56) and the mutation introduction DNA sequence (54) flanked by the pair thereof into the target recombinant gene (40).

More specifically, the Cre-recombinase-mediated reaction of the target recombinant gene (40) and the mutation introduction vector (50) may recombine the mutation target DNA sequence region (35) of the target recombinant gene (40) with the mutation introduction cassette (51) of the mutation introduction vector (50). This recombination may replace the target DNA sequence (32) carried in the mutation target DNA sequence region (35) of the target recombinant gene (40) by the mutant DNA sequence (57) carried in a mutation-introduced DNA sequence region (54) of the mutation introduction vector (50), thereby forming the mutation-introduced gene (60) into which the mutant DNA sequence (57) carrying the gene mutation of interest is introduced. The mediation reaction by the Cre recombinase - mutant lox sequence system may be performed in conventional manner known to the art.

Furthermore, the recombination reaction of recombination of the target recombinant gene (40) with the mutation introduction vector (50) by mediation of the Cre recombinase is also involved in not only the recombination between the target DNA sequence and the mutant DNA sequence, but also the recombination of the first mutant lox sequence (30) of the target recombinant gene (40) with the third mutant lox sequence (52) of the mutation introduction cassette (51) as well as the second mutant lox sequence (36) of the target recombinant gene (40) with the fourth mutant lox sequence (56) of the mutation introduction cassette (51), thereby converting the former and the latter to the fifth mutant lox sequence (64) and the sixth mutant lox sequence (66), respectively.

As shown in Fig. 3, the resulting mutation-introduced gene (60) has a sequence configuration consisting of the first homologous recombination DNA sequence (24), the fifth mutant lox sequence (64), the mutation-introduced DNA sequence region (54), the sixth mutant lox sequence (66), the polyA addition sequence (38), the FRT sequence (39b), and the second homologous recombination DNA sequence (28). Further, the mutation-introduced DNA sequence region (54) in turn has a sequence configuration consisting of the mutant DNA sequence (57), the FRT sequence (39C) and the second positive selection marker DNA sequence (58).

The FRT sequence (39b) and the FRT sequence (39c) may be located in the resulting mutation-introduced gene (60) to remove the drug resistance gene upon production of mice, etc. A male mouse carrying the mutation-introduced gene (60) is intercrossd with a female mouse of wild type, and the born mouse of first filial generation (F1) is then intercrossed with a mouse expressing Flp recombinase to give birth to baby mice in which the DNA sequence region between the FRT sequences has been deleted. Thereafter, the mice so delivered are subjected to passage until this region is no longer present.

A description will now be made regarding the fifth mutant lox sequence (64) and the sixth mutant lox sequence (66) of the mutation-introduced gene (60). As described briefly above, on the one hand, the fifth mutant lox sequence (64) is a mutant lox sequence which is formed by recombination of the first mutant lox sequence (30) of the target recombinant gene (40) with the third mutant lox sequence (52) of the mutation introduction cassette (51). Thus, it has a sequence configuration of both portions of the first and third mutant lox sequences. Therefore, the fifth mutant lox sequence (64) is provided with a mutation in the 5 -pair inverted repeats of the loxP sequence, as in the first mutant lox sequence, and a mutation in the 3 -pair inverted repeats of the loxP sequence, as in the third mutant lox sequence. A combination of the first mutant lox sequence (30) and the second mutant lox sequence (54) may include, for example, lox71/KMR3, etc. In other words, the fifth mutant lox sequence (64) is a mutant lox sequence provided with both mutations of the first mutant lox sequence of the 5 - and 3 -pair inverted repeats of the loxP sequence. The sixth mutant lox sequence (66), on the other hand, is a mutant lox sequence, likewise the fifth mutant lox sequence, which is formed by recombination of the second mutant lox sequence (36) of the target recombinant gene (40) with the fourth mutant lox sequence (56) of the mutation introduction cassette (51). In this sense, the sixth mutant lox sequence has a sequence configuration having both mutations of the second and fourth mutant lox sequences.

The reaction mediated by the Cre recombinase - mutant lox system between the target recombinant gene (40) and the mutation introduction vector (50) may be carried out by conventional methods known to the art.
More specifically, among cells with the target mutation vector introduced therein by conventional vector introduction methods such as electroporation, etc., the homologously recombined cells are selected based on the expression of the marker gene located in the vector. For instance, in the case of the marker gene being a drug resistance gene, the cells of interest can be selected by incubating them in a culture medium containing the drug involved. In other words, for instance, the cells which have underwent neither recombination nor homologous recombination are killed and then removed from the cells with the target recombinant vector introduced therein by suspending them in an appropriate medium such as KSR-GMEM medium, etc., transferring the resulting medium suspension to a G418 medium, and then incubating it. This incubation allows a separation and selection of homologously recombined ES cells from colonies grown and living in the G418 medium. The resulting ES cells may be selected by incubating and subjecting the DNA extracted therefrom to PCR or southern hybridization, etc. Among the methods, the PCR my produce DNA products by amplifying the fragment located between the 3-terminal portion of Neo resistance gene and a partial sequence of the gene KCNQ2 adjacent understream of the 3 -terminus of the short-arm region of the target introduction vector by using a pair of oligonucleotides for amplifying a DNA between the Neo resistance gene and the understream short-arm region. The resulting amplified DNA products are then cleaved with an appropriate restriction enzyme and subjected to southern blotting method using the 3 -terminal region of the short-arm region to form the DNA fragments by target homologous recombination. The clone ES cells are then stored by freeze-drying as accepter ES cells for introduction of a mutation into the gene KCNQ2 for future procedures.

The accepter ES cells produced in the manner as described above may enable an introduction of the gene mutation of the mutation introduction vector into the target gene of a gene such as the gene KCNQ2 by lox-specific recombination using the Cre-mutant lox system. The introduction of the gene mutation into the gene KCNQ2 may be carried out by introducing the mutation introduction vector and a Cre recombinase expression vector in conventional manner using electroporation, etc. By incorporating the mutation introduction vector into the accepter ES cells and infecting the Cre expression vector incorporated into the plasmid with the Cre recombinase gene, the target DNA sequence portion flanked by a pair of the lox sequence factors and carried in the target recombinant vector DNA introduced into the accepter ES cells may be introduced into the gene KCNQ2 in the accepter ES cells by a specific recombination via interaction between the Cre recombinase expressed from the Cre expression vector and the lox sequences.

From the accepter ES cells with the mutation introduction vector introduced therein in the manner as described above, the cells with the gene mutation of interest introduced therein by specific recombination may be selected based on resistance to antibiotics. In the cells having the mutation introduction cassette introduced therein by the Cre-mediated specific recombination, a neomycin resistance gene of the target recombinant vector has been deleted and a peuromycin resistance gene is held instead thereof. Therefore, non-recombined cells are caused to die by incubation in an appropriate culture medium containing antibiotic peuromycin and the cells with the gene mutation introduced therein by the specific recombination can be selected. Colonies of living cells are then separated and incubated, followed by extraction of DNA and confirming the introduction of the mutation by PCR or southern hybridization.

The knock-in non-human mammalian animals according to the present invention may be produced by introducing the ES cells with the mutation-introduced DNA sequence portion inserted therein by recombination into non-mammalian animals of interest, except human. Such animals may include, without limitation to, mice, rats, guinea pigs, hamsters, rabbits, dogs, cats, sheep, pigs, goats, cattles, monkeys, and the like. Rodent animals such as mice, rats, guinea pigs, hamsters, rabbits, etc. are preferred, and mice are particularly preferred.

The ES cells carrying the mutation target DNA sequence portion inserted therein by recombination may be injected into a blastocyst or a 8-cell stage host embryo of a wild-type mouse by the coagulation method or microinjection method to grow up to the blastocyst stage, and then transplanted to the uterus of a foster mother animal, such as a foster mother mouse, etc., in a state of phantom pregnancy, thereby resulting in delivering by sa chimeric animal.

In the case of mice, a female mouse is brought into a state of superovulation by injection with a hormone agent such as PMSG having a FSH-like action or hCG having an LH-like action and then intercrossd with a male mouse. At an appropriate time after fertilization, early embyos such as blastocysts or 8-cell stage host embryos are recollected from the uterus and obiduct thereof. Into the early embyos, the homologously recombined ES cells are injected in vitro to form chimeric embryos.

On the other hand, the phantom-pregnant female mouse to become a foster mother may be produced by intercrossing with a male vasoligated mouse of a normal estrus cycle. To the uterus of the resulting phantom-pregnant female mouse are transplanted chimeric embryos produced in the manner as described above, the mouse is brought pregnant and gives birth to baby mice to produce chimeric mice. In order to secure transplantation of the chimeric embryo and pregnancy, it is preferred that female mice from which fertilized eggs are collected and phantom-pregnant mice becoming foster mothers are produced from a group of female mice having an identical estrus cycle.

The mouse individual having reproductive cells derived from the above ES cells may be confirmed by intercrossing the chimeric mouse with a male mouse of pure line to give birth to an individual of next generation, and the introduction of the ES cells into the germ line of the chimeric mouse is investigated using various phenotypes of the ES cells as markers, which occur in the individuals of next generation. For readiness of confirmation, as a hair color derived from the ES cells appears in the individual of next generation born by intercrossing the above chimeric mouse with the mouse of pure line, it is preferred to confirm the introduction of the ES cells into the germ line using the hair-color of the next generation individual as a marker. For mice, hair colors such as wild mouse color (agouti), black, ocher, chocolate brown, white, etc. are known, however, it is preferred to appropriately select the line of a mouse to be intercrossed with the chimeric mouse by taking the line derived from the ES cells to be used into consideration. It is also possible to make such a selection by extracting DNA from the tail end portion of the mouse and subjecting the DNA to southern blott analysis or PCR assay.

By selecting the animals with the recombined ES cells transplanted to the embryo introduced into the germ line and breeding the chimeric animals, the animals may be produced in which the target gene of interest is expressed. By intercrossing the resulting heterozygous mice with each other, the homozygous mice carrying the target gene of interest introduced therein may be produced. The resulting heterozygotes or the homozygotes carrying the target gene of interest possess the gene mutation stably in all reproductive cells and somatic cells so that the mutation can be conveyed to the descendants efficiently by intercrossing and so on.

A detailed description will be made hereinafter regarding the present invention by way of working examples. It is to be understood herein that the following examples are described solely for illustrative purposes and not for the purpose to limit the present invention to them in any respect, by taking two mutations (i.e., Y284C and A306T) of the KCNQ2 subunit of the voltage-gated potassium channel gene discovered in the human autosomal dominant benign familial neonatal convulsion (BFNC) as an example.

### [EXAMPLE 1]

### (1) A sequence configuration of the target recombinant vector:

The target recombinant vector has a sequence configuration consisting in this order from the 5 -terminus of a plasmid-derived sequence portion, a negative selection marker cassette, a partial sequence of the KCNQ2 gene as the first homologous recombination DNA sequence region (the long-arm region), the lox71 sequence as the first mutant lox sequence, a positive selection marker DNA sequence, the lox2272 sequence as the second mutant lox sequence, the second homologous recombination DNA sequence region (the short-arm region), and a cleavage site by a restriction enzyme for vector linearization.

This target recombinant vector (pTgKCNQ2) was constructed so as to carry a DNA sequence having a full length of 14,164 bp (SQ ID NO:5) using a pBluescript II SK+ plasmid as a base. The plasmid map is as expressed in Fig. 4.
Base Nos. 1 - 673: pBluescript II SK+ derived portion;
Base Nos. 674 - 2301: DT-A cassette;
Base Nos. 2316 - 7483: Partial sequence of the KCNQ2 gene (a long-arm region for homologous recombination);
Base Nos. 7484 - 7517: lox71 sequence;
Base Nos. 8091 - 10138: PGK/Neo cassette;
Base Nos. 10145 - 11939: Partial sequence of the KCNQ2 gene (a short-arm region for homologous recombination);
Base Nos. 11940 - 14164: pBluescript II SK+ derived portion;
Base Nos. 11950 - 11954: restriction enzyme Sac II cleavage site (for vector linearization).

### (1.1) Plasmid:

The plasmid pBluescript II SK+ was linearized by cleaving a closed circular DNA (2,960 bp: Strategene) extracted from Escherichia coli with restriction enzymes Xho I and Xba I.

### (1.2) Negative selection marker cassette (DT-A cassette):

The negative selection marker cassette has a sequence configuration composed of a MC1 promoter, a diphtheria toxin A coding region (DT-A), and a polyA addition sequence (pA).

The plasmid pDT/ApA (distributed by Dr. Kimi Araki) was purified as a 1.6 kb fragment by cleaving the 5 -terminal sequence with Xho I and the 3 -terminal sequence with Spe I.

### (1.3) Long-arm and short-arm regions of the KCNQ2 gene for homologous recombination:

The partial sequences of the gene KCNQ2 structuring the long-arm and short-arm regions for homologous recombination were designed so as to occupy a region ranging from intron 2 to intron 7 of mouse KCNQ2 gene. The long-arm and short-arm regions of the KCNQ2 gene as the target gene were amplified as a 7.4 kb fragment by using a pair of nucleotides for amplifying the long-arm and short-arm regions having the following sequences with a B6 mouse BAC clone RPCI23-401L17 carrying the KCNQ2 gene as a template. The long-arm region corresponds to a region occupying the 5 -terminal 5.6 kb segment, and the short-arm region corresponds to a region occupying the 3 -terminal 1.8 kb segment.

The oligonucleotide pair for amplification of the long-arm and short-arm regions was designed so as to have the base sequences as expressed by SQ ID NO: 6 and NO: 7, respectively.
SQ ID NO:6:
   5-GGGAAGGAGCGGCCGCAGGAAGGGGGTGGAGGGCACTGGACCTG-3 (KQ2-LA5 s)
SQ ID NO:7:
   5-GACGGTGCGCGGCCGCCGTGGCAGCCTGGGAAAGGCCAGAAAGAT-3 (KQ2-SA3 a)
It is noted herein that the underlined portions indicate each the sequence recognizable by the restriction enzyme.

### (1.4) Lox71 sequence as the first mutant lox sequence:

The lox71 sequence as the first mutant lox sequence is a sequence in which a Spe I cleavage site and a complimentarily bondable projection sequence (5 -CTAG-3) are added to both termini of 34 bp double-stranded DNAs corresponding to the loxP sequence. The lox71 sequence was formed by heat denaturing an oligonucleotide pair for synthesizing the lox71 sequence carrying the following sequence at 95°C, base pairing by cooling the reaction mixture to room temperature, and phosphorylating ATP as a phosphoric acid donor to the 5 -termini of the both strands using T4 polynucleotide kinase as a catalyst.

The oligonucleotide pair for synthesizing the lox71 sequence was designed so as to carry the following base sequences as expressed by SQ ID NO:8 and NO:9.
SQ ID NO:8:
   5 -CTAGATAACTTCGTATAATGTATGCTATACGAACGGTA-3 (lox71s)
SQ ID NO:9:
   5 -CTAGITACCGTTCGTATAGCATACATTATACGAAGTTAT-3 (lox71a)
Of the above sequences, the base sequences enclosed by box are base sequences corresponding to the lox71 sequence.

### (1-5) Positive selection marker cassette (Neo^{R} cassette):

The positive selection marker DNA sequence was designed so as to carry the FRT sequence, a PGK/drug resistance gene marker cassette, the lox2272 sequence as the fourth mutant lox sequence, the polyA addition sequence (pA), and the FRT sequence. In this example, neomycin resistance gene (Neo^{R}) was used as a drug resistance gene marker.

The positive selection marker was amplified as a 2.1 kb fragment by amplifying an oligonucleotide pair for amplifying the Neo^{R} cassette having the following sequences by PCR using plasmid p03 (distributed by Dr. Kimi Araki) as a template. Thereafter, the recognition site by a restriction enzyme artificially added to the both termini was cleaved with Nhe I and removed.

The oligonucleotide pair for amplifying the Neo^{R} cassette was designed so as to carry the following sequences as expressed by SQ ID NO: 10 and NO: 11, respectively.
SQ ID NO:10:
5 -TCGAGCTAGCTAATAACTTCGTATAGCATACA-3 (Nh-loxPs)
SQ ID NO:11:
   5 -GAATGCTAGCTTGCATGCCTGCAGGT-3 (Nh-FRTa)
Of the above sequences, the underlined portions correspond to the sites recognizable by the restriction enzymes.

### (2) Method for the production of the target recombinant vector:

After the DT-A cassette produced in Example (1-2) was introduced into the plasmid pBluescript II SK+ cleaved with restriction enzymes Xho I and Xba I in Example (1-1), the long-arm and short-arm region fragments produced in Example (1-3) were introduced into the cleaved site. Further, the fragment with the long-arm and short-arm region fragment introduced therein was then cleaved with Spe I, and the lox71 sequence fragment produced in Example (1-4) was introduced into the cleaved site thereof. Then, the fragment having the lox71 sequence fragment introduced therein was cleaved with Xba I at the position upstream by 200 bp from the 5 -terminus of exon 6 of the long-arm region, followed by introduction of the Neo cassette produced in Example (1-5) into the resulting cleaved site to produce the target recombinant vector. The formation of the target recombinant vector was confirmed by determination of the base sequence thereof. The resulting target recombinant vector was then introduced into ES cells.

### [EXAMPLE 2]

This example is involved in the mutation introduction vector to be used for homologous recombination with the target recombinant vector produced in Example 1.
The KCNQ2 mutation introduction vectors (pMtKCNQ2YC and pMtKCNQ2AT) are two kinds of vectors for introduction of a nucleotide substitution into the KCNQ2 gene of the accepter ES cells by specific recombination so as to convert Tyr²⁸⁴ to Cys or Ala³⁰⁶ to Thr, respectively. The loxKMR3 sequence was located at the 5 -terminus of the KCNQ2 gene fragment (exon 6 and its beforehand and behind portion) of 570 bp carrying each of the above nucleotide substitutions, while the puromycin resistance gene (Puro^{R}) and the lox2272 sequence were located at the 3 - terminus thereof. As these vectors were introduced into the accepter ES cells together with the Cre recombinase expression vector, the region between the lox71 sequence and the lox2272 sequence on the KCNQ gene of the accepter ES cell was substituted by mediation of the Cre recombinase with high efficiency. As the substituted cells become resistant to puromycin, they can be positively selected. Each of the KCNQ2 mutation introduction vectors (pMtKCNQ2YC and pMtKCNQ2AT) is as long as 4,895 bp (SQ ID NO:12), and it has a sequence configuration as will be expressed in plasmid map of Fig. 6.

Base Nos. 1-2243: pBluescript II SK+ derived portion;
Base Nos. 2243-2277: loxKMR3 sequence;

Base Nos. 2278-2847: Partial sequence of gene KCNQ2 (exon 6 carrying mutation and its edge region);
Base Nos. 2848-4243: PGK/Puro cassette; and
Base Nos. 4238-4895: pBluescript II SK+ derived portion.

The configuration of the KCNQ2 gene portion is expressed as follows:
Base Nos. 2278-2471: intron5 (3 -terminal portion);
Base Nos. 2472-2582: exon6 (carrying a mutation);
Base Nos. 2583-2847: intron6 (5 -terminal portion);
Base No. 2506: mutation site (TAC(Tyr)→TGC(Cys) (pMtKCNQ2YC only); and
Base No. 2571: mutation site (GCT(Ala)→ACT(Thr) (pMtKCNQ2AT only).

The PGK/Puro cassette carries the following sequence factors:
Base Nos. 2850-2949: FRT sequence;
Base Nos. 2966-3522: PGK gene promoter region;
Base Nos. 3523-4122: puromycine-N-acetyltransferase coding region; and
Base Nos. 4204-4237: lox2272 sequence.

### (2-1) Plasmid:

The plasmid (p3T) as a base was produced by cleaving a closed circular DNA (3,000 bp; MoBiTec) extracted from Escherichia coli with Hind III and Kpn I and then linearizing it.

### (2-2) Puromycin resistance gene cassette (Puro cassette):

The puromycin resistance gene cassette (Puro cassette) was designed so as to have a sequence configuration composed of FRT sequence - PGK promoter (PPGK) - puromycin resistance gene (Puro^{R})-lox2272 sequence.
The Puro cassette was amplified as a 1.5 kb fragment by PCR using an oligonucleotide pair carrying the following sequence and plasmid p04 (distributed by Dr. Kimi Araki) as a template. Then, the restriction enzyme - recognizable sites artificially added to its 5 - and 3 -termini were cleaved with Hind III and Kpn I.

The oligonucleotide pair for amplifying the Puro cassette was designed to have the following sequences as expressed by SQ ID NO:13 and NO:14.
SQ ID NO:13:
   5 -AACAAGCTTCAAAAGCGCTCTGAAGTTCCTAT-3 (Hd-FRTs)
SQ ID NO:14:
   5 -ATAGGTACCATAACTTCGTATAAAGTATCCTATACGAAGTTA-3 (Kp-lox2272a)
Of the above sequences, the underlined portions are the restriction enzyme - recognizable sequences.

### [EXAMPLE 3]

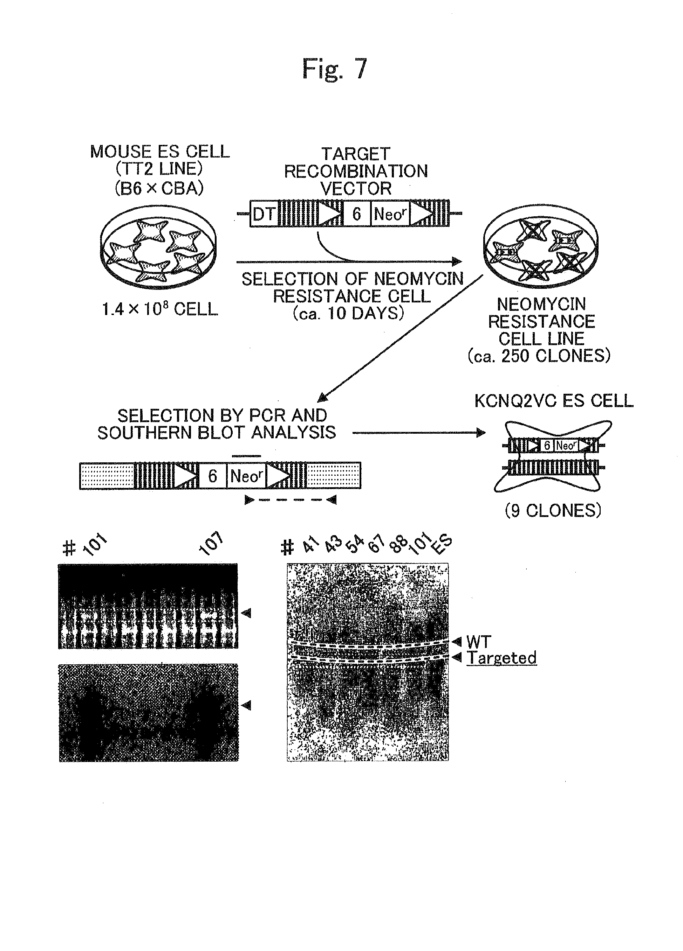

This example is involved in the construction of the accepter ES cells into which a mutation of the KCNQ2 gene can be introduced. The construction of the accepter ES cells may be carred out in the manner as will be described hereinafter (see Fig. 7).
Mouse ES cells were transferred to two sheets of dishes, and the mouse ES cells in a semi-confluent state (a feeder-free KPTU line) were separated from the dish by trypsin digestion, followed by suspending the cells to a total volume of 1.6 ml. To the resulting cells, 20 µg of the linearized target recombinant vector of Example 1 was added, followed by cooling them on ice for 10 minutes and transferring equal amounts to two electroporation cuvettes. Then, the introduction of DNA was carried out by discharging once under the condition of 0.8 kV and 3.0 µF each by a gene pulser (Biorad).
By introducing the DNA of the target recombinant vector into the mouse ES cells by electroporation in the manner as described above, the above sequence factors were introduced into the locus of the KCNQ2 gene on the chromosome by target recombination. The resulting mouse ES cell lines were incubated and selected in a culture medium containing neomycin (G418) so as to allow a survival of only the cells into which the target recombinant vector carrying the neomycin resistance gene (Neo^{R}) was inserted stably in the genomic DNA. In other words, the mouse ES cell lines with the above sequence factors introduced therein were selected by suspending the cells in 60 ml of KSR-GMEM medium after electroporation, transferring equal amounts of the cells to six dishes, exchanging the medium with 200 µg/ml of a G418 medium after 24 hours, and then incubating the cells for 7 days by exchanging the G418 mdia at every 2 days. The non-homologously recombined cells with the target recombinant vector introduced into the position other than the KCNQ2 gene locus were removed by the lethal toxin expressed by the DT-A gene located outside the long-arm region.

Furthermore, the homologously recombined ES cells were isolated and selected from the mouse ES cells which have survived in the G418 medium and formed colonies thereon. As a result of selection of the mouse ES cells by the G418 medium, it was confirmed that a total number of approximately 500 ES cells survived in the G418 medium and formed colonies. Of those cells, 144 cells were isolated and incubated as candidates for cells in which the target homologous recombination have been expected to occur, and DNA was extracted from the cells, followed by selection of the cells by PCR. This PCR was carried out to amplify 2.0 kb fragments between the 3 -terminal portion of the Neo resistance gene and the partial sequence of the KCNQ2 gene adjacent downstream of the 3 -terminus of the short-arm region of the target recombinant vector by using a pair of oligonucleotides for amplifying the sequence between the Neo resistance gene and the downstream portion of the short-arm region. This amplification reaction confirmed amplified products in 9 clones.

The oligonucleotide pair for amplifying the Neo resistance gene and the downstream segment of the short-arm region was designed so as to have the following base sequences as expressed by SQ ID NO:15 and NO:16.
SQ ID NO:15:
   5 -GCAAAACCAAATTCCGGGCCAGCTCATTC-3 (Neo3 s)
SQ ID NO:16:
   5 -ATTTGTCCTGCTTCAGTGCTGTATTGGGAT-3 (KQ2CA3 a)

Then, the DNA of these amplified products was amplified by PCR using a pair of primers having base sequences as will be described hereinafter. As a result, no amplified products were confirmed so that any introduction of the target recombinant vector was not found to have occurred by homologous recombination. In other words, the cleavage of the DNA with restriction enzymes BspH I and Bgl II and the sourthern blot analysis of the 3 -region of the short-arm region using as a probe have detected a 3.2 kb fragment in all 9 clones, which was predicted to be detected in the case of target homologous recombination. This confirmed that the target recombination was expected to occur in the ES cells of 9 clones, and they were stored by freeze-drying as accepter cells capable of introducing the mutation into the KCNQ2 gene for use with further procedures.

The primer pair for amplifying DNA between the DT-A gene for use to detect non-homologous recombination was designed so as to have the following sequences as expressed by SQ ID NO:17 and NO:18:
SQ ID NO:17: 5 -CCTGTGCAGGAAATCGTGTCAGGGCG-3 (DT-A3 s)
SQ ID NO:18: 5 -TTCTCTTCCAGCTGGATCGGGGTGC-3 (KQ2LA5 a)

The primer pair for amplifying the exon 6 edge region and introducing the loxKMR3 sequence was designed so as to have the following sequences as expressed by SQ ID NO:19 and NO:20.
SQ ID NO:19:
   5 -TAGGATCCATAACTTCGTATAGCATACATTATACCTTGTTATCTAGTA-3 (BH-KMR/E6s)
SQ ID NO:20:
   5 -GATAAGCTTAGAATCATTCAGATGGGAAAGCCACA-3 (Hd-E6a)
In the above sequences, the underlined portions indicate sequences recognizable by the restriction enzymes, the portions enclosed by boxes indicate the loxKMR3 sequence, and the letter written in Gothic character indicates the site of mutation substitution.

The primer pair for introducing the mutation Y284C was designed so as to have the following base sequence as expressed by SQ ID NO:21 and NO:22.
SQ ID NO:21:
   5 -CGACCATTGGCTACGGGGACAAGTGCCCTCA-3 (Y284Cs)
SQ ID NO:22:
   5 -GCCTCCCGTTCCAGGTCTGAGGGCACTTGT-3 (Y284Ca)

The primer pair for introducing the mutation A306T was designed so as to have the following base sequence as expressed by SQ ID NO:23 and NO:24.
SQ ID NO:23:
   5 -CCCTCAthe target geneTGTCTCGTTCTTTACTCTTC-3 (A306Ts)
SQ ID NO:24:
   5 -TCCCAAAATGCCAGCAGGAAGAGTAAAGAA-3 (A306Ta)

### [EXAMPLE 4]

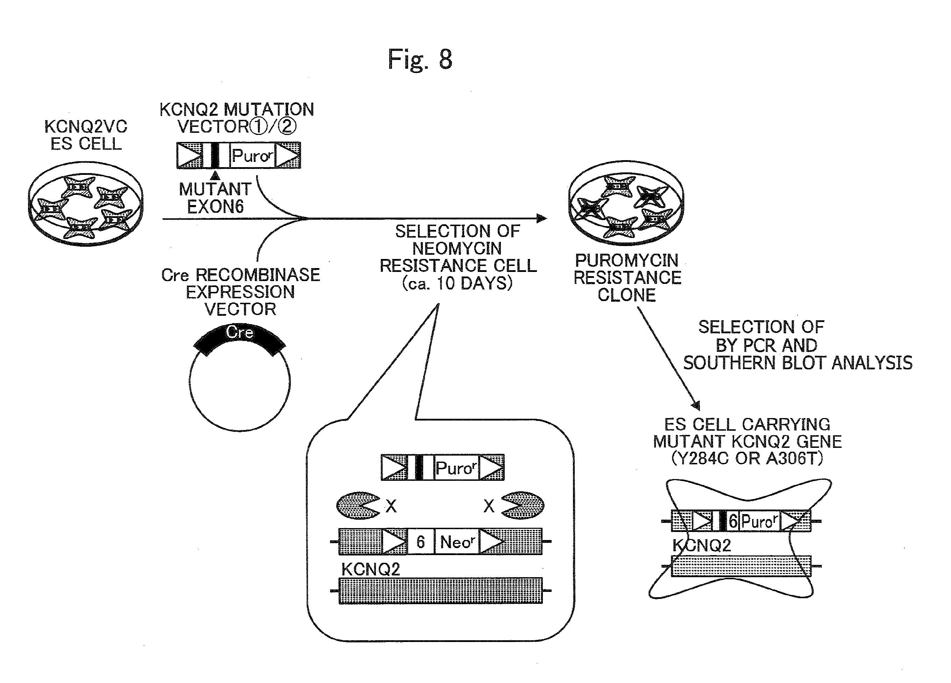

Furthermore, the mutation introduction vector produced in Example 2 and the Cre recombinase expression vector were introduced into the clone of the mutant accepter ES cell line produced and isolated in Example 3 by electroporation under conditions of 400 V and 125 µF, thereby confirming the introduction of the mutant sequence between the two lox sequences of the KCNQ2 gene on the chromosome and the selection and isolatation of the substituted mutant ES cells by the puromycin resistance gene. In other words, the electroporated ES cells were suspended in 60 ml of a KSR-GMEM medium, and equal amounts of the suspension were transferred to six 10-cm dishes, followed by incubation and exchange for 24 µg/ml of a puromycin medium after 24 hours. Thereafter, the puromycin medium was exchanged at every 2 days and the incubation was continued for 7 days. It was then confirmed that only the cells having the region between the two lox sequences replaced by the mutant gene sequence formed colonies (see Fig. 8).

Thereafter, the ES cells having the KCNQ2 mutation were isolated and confirmed. Plural puromycin resistance cell lines with colonies formed in the above manner were isolated and stored by freeze-drying as candidates for the KCNQ2 mutant ES cells. The DNA was extracted from a portion of those cells and the region carrying the mutation was amplified by PCR, followed by confirmation of the mutation introduction by determination of the base sequence.

### [EXAMPLE 5]

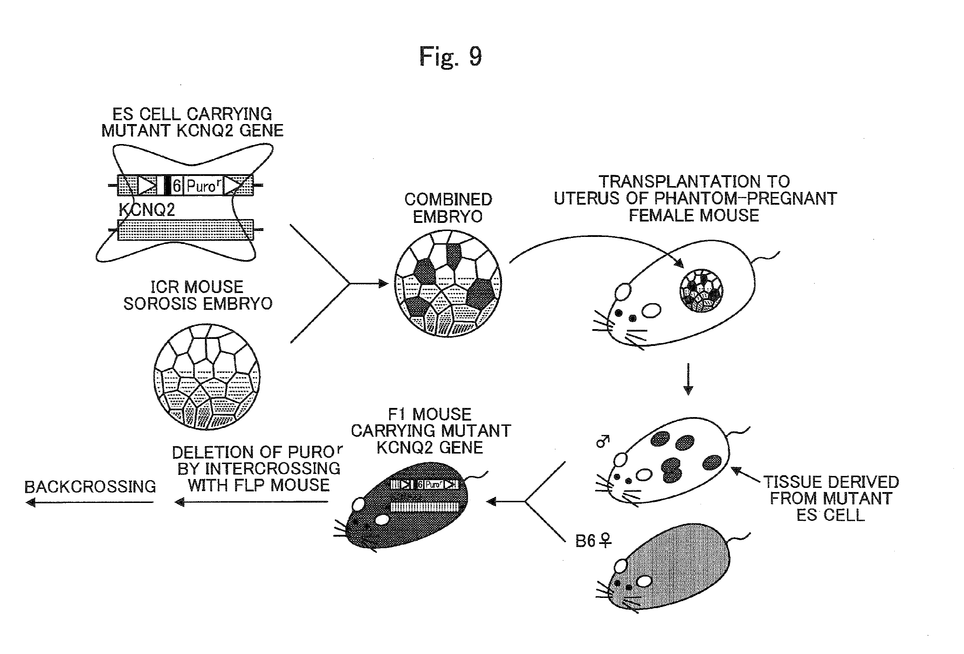

Knock-in mice were produced by injecting the KCNQ2 mutation-introduced ES cells established in the above manner into mice in conventional techniques known to the art. In other words, the KCNQ2 mutation-introduced ES cell clones were coagulated with the sorosis embryo from an ICR mouse and incubated overnight, followed by selection of a mixed embryo in which the ES cell was coagulated with the sorosis embryo. This chimeric embryo was then transplanted to the uterus of a phanthom-pregnant female mouse (i.e., a foster mother). Of mice delivered in about 17 days, the mice having a specific hair color originating from the tissue derived from the KCNQ2 cells were selected as chimeric mice. After the chimeric mice were sexually matured in about 8 weeks after birth, they were intercrossd with C57BL/6 mice to give birth to F1 mice (heterozygotes) having a mosaic hair color originating from the KCNQ2 mutant cells derived from the ES clone. The F1 mice were then intercrossd with Flp expression mice to remove the unnecessary sequence factors such as the puromycin resistance gene, etc., flanked by the FRT sequences, resulting in the formation of knock-in mice having a desired mutation carrying the mutation Y284C or A306T in the gene KCNQ2 and the other DNA sequence substantially identical to the DNA sequence of a wild-type mouse (see Fig. 9).

### INDUSTRIAL APPLICABILITY

The mutation introduction vector of a variable type according to the present invention can be applied to any mutation of genes and to permit a rapid formation of knock-in non-humam mammals such as knock-in mice carrying the mutant gene with the desired gene mutation introduced therein. In addition, the knock-in non-human mammalian animals according to the present invention can be expected to greatly contribute to research and review on clarification, etc. of causes and outbreaks of diseases and the like, particularly to the medical field.

### [Explanation of Reference Numbers & Symbols]

10 target gene
10a first DNA sequence region
12 mutation introduction exon
20 target recombinant vector (targeting vector)
20a second DNA sequence region
21 negative selection marker DNA sequence region
24 first homologous recombination DNA sequence region
25 target DNA sequence region
28 second homologous recombination DNA sequence region
29 first mutant lox sequence
32 target DNA sequence
33 first positive selection marker DNA sequence region
34 mutation target DNA sequence region
35 second mutant lox sequence
38 PolyA addition sequence
39a FRT sequence
39b FRT sequence
39c FRT sequence
39 target recombinant gene
40a third DNA sequence region
50 mutation introduction vector
51 mutation introduction cassette
52 third mutant lox sequence
53 mutation-introduced DNA sequence region
56 fourth mutant lox sequence
57 second positive selection marker DNA sequence region
60 mutation-introduced gene
64 fifth mutant lox sequence
66 sixth mutant lox sequence

## Claims

1. A method for the introduction of a gene mutation wherein a target DNA sequence (32) contained in a target recombinant gene (40) on a chromosome and flanked by a pair of a first mutant lox sequence (30) and a second mutant lox sequence (36) different therefrom is recombined with a mutation-introduced DNA sequence region (54) contained in a mutation introduction cassette (51) of a mutation introduction vector (50) and flanked by a pair of a third mutant lox sequence (52) and a fourth mutant lox sequence (56) different therefrom via the mutation introduction cassette (51) of the mutation introduction vector (50) by mediation of a Cre recombinase.

2. The method for introducing the gene mutation as claimed in claim 1, wherein the target recombinant gene (40) is a recombinant gene produced by homologous recombination of the target gene (10) and the target recombinant vector (20) and carries a target DNA sequence (32) flanked by a pair of the first mutant lox sequence (30) and the second mutant lox sequence (36) different therefrom.

3. The method for introducing the gene mutation as claimed in claim 1 or 2, wherein the mutation introduction cassette (51) of the mutation introduction vector (50) carries a mutant DNA sequence (57) and a second positive selection marker DNA sequence (58), flanked by a pair of the third mutant lox sequence (52) and the fourth mutant lox sequence (56).

4. The method for introducing the gene mutation as claimed in any one of claims 1 to 3, wherein the mutant DNA sequence (57) carried in the mutation introduction cassette (51) of the mutation introduction vector (50) and flanked by a pair of the third mutant lox sequence (52) and the fourth mutant lox sequence (56) is recombined with the target DNA sequence (32) carried in the target recombinant gene (40) and flanked by a pair of the first mutant lox sequence (30) and the second mutant lox sequence (36) by mediation of Cre recombinase.

5. The method for introducing the gene mutation as claimed in any one of claims 1 to 4, wherein the first mutant lox sequence (30) is a mutant lox sequence carrying a mutation in 5 -pair inverted repeats of loxP sequence, the second mutant lox sequence (36) is a mutant lox sequence carrying a mutation in spacer sequence of loxP sequence, the third mutant lox sequence (52) is a mutant lox sequence carrying a mutation in a 3 - pair inverted repeats of loxP sequence, and the fourth mutant lox sequence (56) is a mutant lox sequence carrying a mutation in spacer sequence of loxP sequence.

6. The method for introducing the gene mutation as claimed in any one of claims 1 to 5, wherein the first mutant lox sequence (30) is lox71 sequence, the second mutant lox sequence (36) is lox2272 sequence, the third mutant lox sequence (52) is loxKMR3 sequence, and the fourth mutant lox sequence (56) is lox2272.

7. The method for introducing the gene mutation as claimed in any one of claims 1 to 6, wherein the mutation-introduced gene (60) produced by recombination of the target recombinant gene (40) and the mutation introduction cassette (51) of the mutation introduction vector (50) carries the mutation-introduced DNA sequence region (54) which is flanked by a pair of the fifth mutant lox sequence (64) and the sixth mutant lox sequence (66) formed by recombination of a pair of the first mutant lox sequence (30) and the second mutant lox sequence (36) with a pair of the third mutant lox sequence (52) and the fourth mutant lox sequence (56) and which in turn carries the mutant DNA sequence (57) and the second positive selection marker DNA sequence (58).

8. The method for introducing the gene mutation as claimed in claim 7, wherein the fifth mutant lox sequence (62) is lox71/KMR3 carrying a mutation of lox71 sequence which is the first mutant lox sequence (30) and a mutation of loxKMR3 sequence which the third mutant lox sequence (36) in the 5 - and 3 -pair inverted repeats, respectively, and the sixth mutant lox sequence (66) is lox2272.

9. The method for introducing the gene mutation as claimed in any one of claims 1 to 8, comprising a homologous recombination step of homologous recombination of the target gene (10) with the target recombinant vector (20) to homologously recombine a first DNA sequence region (10a) of the target gene (10) with a second DNA sequence region (20a) of the target recombinant vector, thereby recombining a mutation introduction exon (12) carried in the first DNA sequence region (10a) with the target DNA sequence (30) carried in the second DNA sequence region (20a) to form the target recombinant gene (40); and
a mutation introduction step of introducing the mutation-introduced DNA sequence region (54) to form a mutation-introduced gene (60) by recombining a pair of the first mutant lox sequence (30) and the second mutant lox sequence (36), carried in a third DNA sequence region (40a) of the target recombinant gene (40), and the mutation target DNA sequence region (35) flanked by the pair thereof, with the mutation introduction cassette (51) of the mutation introduction vector (50) by mediation of a Cre - mutant lox system composed of Cre recombinase and a pair of mutant lox sequences and introducing the mutation-introduced DNA sequence region (54);
wherein the target recombinant vector (20) comprises a negative selection marker DNA sequence (22), a first homologous recombination DNA sequence region (24), a target DNA sequence region (26) and a second homologous recombination DNA sequence region (34), the target DNA sequence region (26) comprises the first mutant lox sequence (30), the target DNA sequence (32), a first positive selection marker DNA sequence (34), the second mutant lox sequence (36) and a polyA addition sequence (38), and the first and second homologous recombination DNA sequence regions (22, 28) have each a length of the DNA sequence necessary for homologous recombination;
wherein the third DNA sequence region (40a) of the target recombinant gene (40) comprises the first homologous recombination DNA sequence region (24), the first mutant lox sequence (30), the mutation target DNA sequences region (35), the second mutant lox sequence (36) and the second homologous recombination DNA sequence region (28), and the mutation target DNA sequence region (35) comprises the target DNA sequence (32) and the first positive selection marker DNA sequence (34);
wherein the mutation introduction cassette (51) of the mutation introduction vector (50) comprises the third mutant lox sequence (52), the mutation-introduced DNA sequence region (54) and the fourth mutant lox sequence (56), and the mutation-introduced DNA sequence region (54) comprises a mutant DNA sequence (57) and the second positive selection marker DNA sequence (58); and
wherein the mutation-introduced gene (60) comprises the first homologous recombination DNA sequence region (24), the fifth mutant lox sequence (62), the mutation-introduced DNA sequence region (54), the sixth mutant lox sequence (66) and the second homologous recombination DNA sequence region (28), and the mutation-introduced DNA sequence region (54) comprises the mutant DNA sequence (57) and the second positive selection marker DNA sequence (58).

10. The method for introducing the gene mutation as claimed in any one of claims 1 to 9, wherein the mutant DNA sequence (57) carried in the mutation introduction cassette (51) of the mutation introduction vector (50) is a mutant exon.

11. The method for introducing the gene mutation as claimed in any one of claims 1 to 10, wherein the mutant DNA sequence (57) carried in the mutation-introduced gene (60) is a mutant exon.

12. The method for introducing the gene mutation as claimed in any one of claims 1 to 11, wherein the target gene is a gene derived from a KCNQ2 subunit of voltage-gated potassium channel gene associated with human autosomal dominant benign familial neonatal convulsion (BFNC).

13. The method for introducing the gene mutation as claimed in any one of claims 1 to 12, wherein the target mutation of the target gene (10) is a mutation Y284C (SQ ID NO: 1 and NO:2) or A306T (SQ ID NO:3 and NO:4) of gene KCNQ2.

14. A method for the production of a mutation-introduced gene comprising producing a mutation-introduced gene (60) by introducing a gene mutation into the target gene (10) by the method for introducing the gene mutation as claimed in any one of claims 1 to 13.

15. A mutation-introduced gene **characterized in that** the mutation-introduced gene comprises a mutation-introduced gene (60) carrying a first homologous recombination DNA sequence region (24), a fifth mutant lox sequence (62), a mutation-introduced DNA sequence region (54), a sixth mutant lox sequence (66) and a second homologous recombination DNA sequence region (28), wherein the mutant DNA sequence region (54) comprises a mutant DNA sequence (57) and a second positive selection marker DNA sequence (58), which are flanked by a pair of the fifth mutant lox sequence (62) and the sixth mutant lox sequence (66).

16. The mutation-introduced gene as claimed in claim 15, wherein a pair of the fifth mutant lox sequence (62) and the sixth mutant lox sequence (66) is formed by recombination of a pair of the first mutant lox sequence (30) and the second mutant lox sequence (36) with a pair of the third mutant lox sequence (52) and the fourth mutant lox sequence (56).

17. A mutation introduction cassette **characterized in that** the mutation introduction cassette comprises a pair of the third mutant lox sequence (52) and the fourth mutant lox sequence (56) as well as the mutant DNA sequence (57) and a second positive selection marker DNA sequence (58) flanked by the pair thereof.

18. The mutation introduction cassette as claimed in claim 17, wherein the third mutant lox sequence (52) is loxKMR33 sequence and the fourth mutant lox sequence (56) is lox2272, and the mutant DNA sequence carries mutant exon.

19. A mutation introduction vector caracterized in that the mutation introduction vector carries the mutation introduction cassette as claimed in claim 17 or 18.

20. A mutant ES cell **characterized in that** the mutation-introduced gene produced by the method for introducing the gene mutation as claimed in claim 14 or the mutation-introduced gene as claimed in claim 15 or 16 is introduced.

21. A knock-in non-human mammalian animal **characterized in that** the mutation-introduced gene produced by the method for introducing the gene mutation as claimed in claim 14 or the mutation-introduced gene as claimed in claim 15 or 16 is introduced.

22. The knock-in non-human mammalian animal as claimed in claim 21, wherein the knock-in non-human mammalian animal is a mouse.

23. The knock-in non-human mammalian animal as claimed in claim 21 or 22, wherein the mutant gene is a mutant gene derived from KCNQ2 subunit of voltage-gated potassium channel gene associated with human autosomal dominant benign familial neonatal convulsion (BFNC).

24. The knock-in non-human mammalian animal as claimed in any one of claims 18 to 23, wherein the mutant gene is a mutation Y284C or A306T of the mutant gene KCNQ2.


SEQUENCE LISTING

<110>  Fukuoka University
       Kumamoto University

<120>  Mutation-introduced gene and knock-in non-human animals with same

<130>  NP08234-YS

<160>  24

<170>  PatentIn version 3.1

<210>  1
<211>  111
<212>  DNA
<213>  Artificial sequence

<220>
<223>  Mutated DNA of exon 6 in KCNQ2 gene(Homo sapience)

<400>  1
atcaccctga cgaccattgg ctgcggggac aagtaccctc agacctggaa cgggaggctg      60

ctggcagcga cctttaccct cattggtgtc tcgttctttg ctcttcctgc t              111


<210>  2
<211>  37
<212>  PRT
<213>  Artificial sequence

<220>
<223>  Peptide encoded by SEQ ID NO: 1

<400>  2

Ile Thr Leu Thr Thr Ile Gly Cys Gly Asp Lys Tyr Pro Gln Thr Trp
1               5                   10                  15


Asn Gly Arg Leu Leu Ala Ala Thr Phe Thr Leu Ile Gly Val Ser Phe
            20                  25                  30


Phe Ala Leu Pro Ala
        35


<210>  3
<211>  111
<212>  DNA
<213>  Artificial sequence

<220>
<223>  Mutated DNA of exon 6 in KCNQ2 gene(Homo sapience)

<400>  3
atcaccctga cgaccattgg ctacggggac aagtgccctc agacctggaa cgggaggctg      60

ctggcagcga cctttaccct cattggtgtc tcgttcttta ctcttcctgc t              111


<210>  4
<211>  37
<212>  PRT
<213>  Artificial sequence

<220>
<223>  Peptide encoded by SEQ ID NO: 3

<400>  4

Ile Thr Leu Thr Thr Ile Gly Tyr Gly Asp Lys Cys Pro Gln Thr Trp
1               5                   10                  15

Asn Gly Arg Leu Leu Ala Ala Thr Phe Thr Leu Ile Gly Val Ser Phe
            20                  25                  30

Phe Thr Leu Pro Ala
        35

<210>  5
<211>  14164
<212>  DNA
<213>  plasmid

<400>  5
ctaaattgta agcgttaata ttttgttaaa attcgcgtta aatttttgtt aaatcagctc      60

attttttaac caataggccg aaatcggcaa aatcccttat aaatcaaaag aatagaccga      120

gatagggttg agtgttgttc cagtttggaa caagagtcca ctattaaaga acgtggactc      180

caacgtcaaa gggcgaaaaa ccgtctatca gggcgatggc ccactacgtg aaccatcacc      240

ctaatcaagt tttttggggt cgaggtgccg taaagcacta aatcggaacc ctaaagggag      300

cccccgattt agagcttgac ggggaaagcc ggcgaacgtg gcgagaaagg aagggaagaa      360

agcgaaagga gcgggcgcta gggcgctggc aagtgtagcg gtcacgctgc gcgtaaccac      420

cacacccgcc gcgcttaatg cgccgctaca gggcgcgtcc cattcgccat tcaggctgcg      480

caactgttgg gaagggcgat cggtgcgggc ctcttcgcta ttacgccagc tggcgaaagg      540

gggatgtgct gcaaggcgat taagttgggt aacgccaggg ttttcccagt cacgacgttg      600

taaaacgacg gccagtgagc gcgcgtaata cgactcacta tagggcgaat tgggtaccgg      660

gccccccctc gagcagtgtg gttttcaaga ggaagcaaaa agcctctcca cccaggcctg      720

gaatgtttcc acccaatgtc gagcagtgtg gttttgcaag aggaagcaaa aagcctctcc      780

acccaggcct ggaatgtttc cacccaatgt cgagcaaacc ccgcccagcg tcttgtcatt      840

ggcgaattcg aacacgcaga tgcagtcggg gcggcgcggt cccaggtcca cttcgcatat      900

taaggtgacg cgtgtggcct cgaacaccga gcgaccctgc aggtcctcgc catggatcct      960

gatgatgttg ttgattcttc taaatctttt gtgatggaaa acttttcttc gtaccacggg      1020

actaaacctg gttatgtaga ttccattcaa aaaggtatac aaaagccaaa atctggtaca      1080

caaggaaatt atgacgatga ttggaaaggg ttttatagta ccgacaataa atacgacgct      1140

gcgggatact ctgtagataa tgaaaacccg ctctctggaa aagctggagg cgtggtcaaa      1200

gtgacgtatc caggactgac gaaggttctc gcactaaaag tggataatgc cgaaactatt      1260

aagaaagagt taggtttaag tctcactgaa ccgttgatgg agcaagtcgg aacggaagag      1320

tttatcaaaa ggttcggtga tggtgcttcg cgtgtagtgc tcagccttcc cttcgctgag 1380
gggagttcta gcgttgaata tattaataac tgggaacagg cgaaagcgtt aagcgtagaa 1440
cttgagatta attttgaaac ccgtggaaaa cgtggccaag atgcgatgta tgagtatatg 1500
gctcaagcct gtgcaggaaa tcgtgtcagg cgatctcttt gtgaaggaac cttacttctg 1560
tggtgtgaca taattggaca aactacctac agagatttaa agctctaagg taaatataaa 1620
atttttaagt gtataatgtg ttaaactact gattctaatt gtttgtgtat tttagattcc 1680
aacctatgga actgatgaat gggagcagtg gtggaatgca gatccactag agtcgaagcg 1740
gcttcactcc tcaggtgcag gctgcctatc agaaggtggt ggctggtgtg gccaatgccc 1800
tggctcacaa ataccactga gatctttttc cctctgccaa aaattatggg gacatcatga 1860
agccccttga gcatctgact tctggctaat aaaggaaatt tattttcatt gcaatagtgt 1920
gttggaattt tttgtgtctc tcactcggaa ggacatatgg gaagggcaaa tcatttaaaa 1980
catcagaatg agtatttggt ttagagtttg gcaacatatg cccatatgct ggctgccatg 2040
aacaaaggtt ggctataaag aggtcatcag tatatgaaac agccccctgc tgtccattcc 2100
ttattccata gaaaagcctt gacttgaggt tagatttttt ttatattttg ttttgtgtta 2160
tttttttctt taacatccct aaaattttcc ttacatgttt tactagccag atttttcctc 2220
ctctcctgac tactcccagt catagctgtc cctcttctct tatggagatc cctcgacctg 2280
cagcccgggg gatcctgatc cactagagcg gccgccgtgg cagcctggga aaggccagaa 2340
agataatctg agttcttgat cagcaccccg atccagctgg aagagaattt ttctgcctct 2400
gaacctagga aggaattgtt gcataccagg aaaaataatg aatatttacc ttctgtaggg 2460
caggcagcgt ggagttggtt ctttttattg tttattgttt tttcactagc taaggctgcc 2520
tggggtaaaa ccagacaagg atacagaaag gctggtcagc aatcagtgtg actgtggagt 2580
cttgggtagc agaaaacagg gtgtggactg gccccctgct atgctctgag ggccctctaa 2640
ggtctcaagt tctaggcatt ttagctaata ggcacgggct gcactgacca tggctggccc 2700
actgactgcg tgtctctcac cttctgcagg ttccttttag tcttctcctg ccttgtgctt 2760
tctgtgtttt ccaccatcaa ggagtacgag aagagctctg agggggccct ctacatcttg 2820
gtaagcctca cacttgggca gggcatgggg agaggcctgg aggctgggaa atggacctct 2880
gcagcagtct gcctcccaac cccggacctc tgcttctgct tctgccctca accctgatcg 2940
tcggagtgag ccaggccttc tgcatacacg gtctcctcca gagtagttag tgttctttct 3000
tgcacacaca gctactgttg ctcccagcat tgtggtgtag agacctcaag tcccttccca 3060
gtccttctcc tagtccctag aagtgatata acaacaccca gcagttctca ccactccccc 3120
ctccctttcc gccctgccct ctggagtctc cgtgctcagg tgcccccact gaagtatgtg 3180
gtgctcccca gaagatgtgg gtgaaggggt gggaggccat gatgctcgct catataaacc 3240
ctgtgacggg acaggggtgg gtgtcatgga aacgggactg taattcctct gttgccattg 3300
gtaacggaga tggcccttca agaatgatcc cactagagtg cttttccacc cactttgccc 3360

cactgccctg gagctgcctg aattctaaag gaacctgttc tccttttggt atggctggga 3420
agtctctcag gtcagctctg ctgtgcccac ccctcacact gacactagag cctcccaatc 3480
cagctcagaa gggcggtgag aaggggggtt cctctggtgg tctacaggcc aggacttgct 3540
ggcagcagta agtccctgga gggtctgttt ctagcaggga ttcaatcctg gctgacatga 3600
gctaggtgcc ctgtggggtt tgagctaagc ctgaaggtct gatttctagc atctgatccc 3660
tggggcttta tccatccctg tcccgaaagt gtcatgtagt atagaggaag ccctcctaag 3720
gcctgatagt gtcctcatcc gtgttcctcc cacctcccag gaaatcgtga ctatcgtggt 3780
attcggtgtt gagtactttg tgaggatctg ggctgcaggc tgctgttgcc ggtatcgagg 3840
ctggaggggc aggctcaagt ttgccaggaa gccgttctgt gtgattggtg aggcctggtg 3900
gacatgccca ccagagttgg gtctggggag agagttgctt ctgtcttgtc cataatgggc 3960
tgagtgagag ggtagggcct tctggaagtt ccttcttatc accgacttgg agttggaggt 4020
gcaatggctg acagggccta ggtcttggga gggagacaca gtactgggca gtctctcctg 4080
gacactacag gtaggcaggg tgggcactgt aaaccaccgt tcccagggcc ccacattacc 4140
ttgcatgtct acggggccat tgagtcatgc tctgtggacc ccggggagga gatgctgtga 4200
tctgtgcctg tctcccacag atatcatggt gctgattgcc tccattgctg tgctggctgc 4260
tggttcccag ggcaatgtct ttgccacatc tgcgcttcgg agcttgcggt tcttgcaaat 4320
cttgcggatg atccgtatgg accggagggg tggcacctgg aagctcttgg gatcggtagt 4380
ctacgctcac agcaaggtga gcagcactga cccagcctgg agcctgctgt ttaccatgac 4440
ttcaaggctc caggctgctc taagctgaag tagtcatggg ccaggcaaga tataaatgag 4500
cctctccttt tatcccatgg ctgactcttc ctcacttgtc ctcagaagct attttctatt 4560
cttagtgtga gcaatccttc ctggagctgg cagcccctgt agctgagcat ttcccttcct 4620
ggttctgagg gcctggatcg atactctgcc tgaatcacct gcttagggtt tgtttaagct 4680
gtggcttatg gtggctgcag accagggtac ttgtggggtg gctgtgggaa cacatttctg 4740
tgttgaaaga gtcctcagtg gccaagacct gatttctgcc atgttgtcta tgggattttt 4800
gtgctcttta tgttctcact gactcaaatg ctctctatcc tacctgtccc ccttttgtca 4860
gccatggtct tgtgggggca ggcactgggc tctggggaag ttgacctccc tagaggtctg 4920
gtctctccct gtctctgcac tgcacaccca gagtgttggg aactgagtat acgcctgttg 4980
ggacctgtac cccttcttgg ggatatctcc acagtgctgg gaccccagct gctggctgtg 5040
gggtatgtgt ttccccagcc atggacaggg tttttaaagg actgctgata ctcttctccc 5100
aggtctgaaa tagtcagttg aaagttgcat tgtacagccg gcagtcactt cattatccag 5160
gacaagctct gttgccatcc ctgaccagtg tctgctctaa gttgttcctt gctgccatcg 5220
ggaggggatt ccatcctcca agtccccgag ctgcacagtt gagggcaggg ctcccatgct 5280
gggcttccag gctgacctga gagagacaat gacgtggctt cctgcctttg caggagctgg 5340
tgactgcctg gtacattggc ttcctctgcc tcatcctggc ctcatttctg gtgtacttgg 5400

cagaaaaggg tgagaatgac cactttgaca cctacgcaga tgcactctgg tggggtctgg 5460
taagtcctgg tcactggtca ccattccttt ctcctccaca tcctctggtt actgctatcc 5520
taaggatata taccatgccc cttgggacct gaaggagaga cctagctgtc tttaggctgg 5580
gctgggtggg aggacacctg gtggctggct gtggacataa tgacaactga ggctgatgtg 5640
cttctagtgt tggtagatgt gacagactgg gttcctttct cccccttact acctgcaatt 5700
ccccacttgg tgatggactg tgaaggagaa agattagggt gaaggaatat gggctctgag 5760
agtaatgacc agaatagagc catcctaggg ctccccagct ccggagtttg aatgaccctc 5820
ctaactttgg agcagtgtga gggtggaggt aacattgtta tcttggcctt ccattcccaa 5880
gaccaactgc caccattcca ggtacttgct ggccttccgt ctccagtcaa tcctgccttt 5940
gattgtgagg gacaagggag agttcaaagt gaagatggct cagtgggtaa agtacttgct 6000
tcgaaagcat tgagatttca gtttggatct ccaggagccg tgtagaaatt aggtacatgt 6060
gtgtgtgcac acacagacac acacagacac acagacatac acacacacac acagacacac 6120
agacacacac acacacacac acacacacac acacacacac acacacagca aatggaaaac 6180
acaggccctg actcactctc cgggatgttt tttaggtggt ctgcagcccg ctgacagtga 6240
cttaattgaa gcttccccaa gacttgctgc tgcccaagtg gcttttgtgc ctgaggctcc 6300
ccatcctgac tctgcctagg acactccaga agtcctttct cactgcccct ttccagaagg 6360
gcctactggt tatctttgaa cttatgcttg aagtggacat ggaagacagg tttcctaggc 6420
ccctgttttc tgatcttgct ttctggagcc cacactagcc tcgagaccta ctccaatgag 6480
atagaaatgg gtttttccta gtccctcccc caatgcttta gactccttgg aggctcaggc 6540
agaggctgtt gtcacttgac aagccacgag gtgatgacct aggcaggtgc tgtgcttgca 6600
gagtcagggg ggagggttag acaactgccc ctagggtgaa gccacagggc agtttatttg 6660
agatcttact aatgcccaca ggtctggaac cctgtgccct tttgggaaga agcagggcta 6720
ttgggagtga cgggagttta agactggaga tgactggtga ggagattgtg tgattacagg 6780
gcaggagcag ctggattgtc tgagtgtgta tgcccaggct ctgctgaggg aggaagcttg 6840
gtaccctggg gtcctgaatt ctaaccagct ggccccaggg ttctgtgaga gcgatgtagc 6900
ccattctttt cagaatgttc tcgtgggatg gggggggggc ggggagggta ggccttcctt 6960
ctctttcaga actgaagcaa acattttatc tttattgttt caaagtaatc cttgtatttt 7020
caaatgtcca tgggtttcta aaatgcagcc ccctcgctac tacccatctg tcttcttggc 7080
taccctctac ccaccacccc ttacctcctc ccctcctggg cccaaccatc tcctagattg 7140
tcccctattc tccctgaatc ctgctctcct aggcctttct gactcctaac actctgtgga 7200
cctggaatgc ttgcacttcc ttgatcttgt cccctgactt ggtaggcacg tactggttaa 7260
ctggtgcccc ttcctctgag cacagagcag gggctagaaa tgccagcaac tcatctggat 7320
gaaaactctc cttgcttggt gccctatgca aacagtgtag tttatcctct catgtgtgaa 7380
ttaggggagc cttgggaatg gttccccttt tagtgtagaa ttaagattgg ggattgtaga 7440

ataaaggaaa gcctatgctg actctggcct tgtttggcac tagtaccgtt cgtatagcat 7500
acattatacg aagttatcta gtatggatga tgaaggaagg atgacctcct gtaggtacag 7560
ctgaagtctg aatgaccatc agtgtccagc ttcccctaaa ctacttggat gaacttccca 7620
tggctgagca gggacatctg gcctgggatt gcgacctctg tggtaggcca tttccagcct 7680
gcccaagtaa ccagagcccc ttacccctca gatcaccctg acgaccattg gctacgggga 7740
caagtaccct cagacctgga acgggaggct gctggcagcg acctttaccc tcattggtgt 7800
ctcgttcttt gctcttcctg ctgtgagtcc cgcgcacctt cctactctgg agatgttagg 7860
ggttttagag gctccccata aggcttggcc atggcccacc tgtgaactgt gaacttggac 7920
acagtcctgt ggggtttaca gaccctagag agctccagac tagaccagag tgaggctgct 7980
ctggtttaga tgcctgcggc ctccctaggc acacttctag tgtcttcaga tcaggccttc 8040
tcacatagct ctgttgattt ctgtggcttt cccatctgaa tgattctagc taataacttc 8100
gtatagcata cattatacga agttatatta agggttccgg atccgatgat atcagatccc 8160
catcaagctt caaaagcgct ctgaagttcc tatactttct agagaatagg aacttcggaa 8220
taggaacttc aagatccact agcgataagc tgcccccgac ctcgacgaat tcatcgatga 8280
ttcgacctcg aaattctacc gggtagggga ggcgcttttc ccaaggcagt ctggagcatg 8340
cgctttagca gccccgctgg gcacttggcg ctacacaagt ggcctctggc ctcgcacaca 8400
ttccacatcc accggtaggc gccaaccggc tccgttcttt ggtggcccct tcgcgccacc 8460
ttctactcct cccctagtca ggaagttccc ccccgccccg cagctcgcgt cgtgcaggac 8520
gtgacaaatg gaagtagcac gtctcactag tctcgtgcag atggacagca ccgctgagca 8580
atggaagcgg gtaggccttt ggggcagcgg ccaatagcag ctttgctcct tcgctttctg 8640
ggctcagagg ctgggaaggg gtgggtccgg gggcgggctc aggggcgggc tcaggggcgg 8700
ggcgggcgcc cgaaggtcct ccggaggccc ggcattctcg cacgcttcaa aagcgcacgt 8760
ctgccgcgct gttctcctct tcctcatctc cgggcctttc gacctgcagc caatatggga 8820
tcggccattg aacaagatgg attgcacgca ggttctccgg ccgcttgggt ggagaggcta 8880
ttcggctatg actgggcaca acagacaatc ggctgctctg atgccgccgt gttccggctg 8940
tcagcgcagg ggcgcccggt tctttttgtc aagaccgacc tgtccggtgc cctgaatgaa 9000
ctgcaggacg aggcagcgcg gctatcgtgg ctggccacga cgggcgttcc ttgcgcagct 9060
gtgctcgacg ttgtcactga agcgggaagg gactggctgc tattgggcga agtgccgggg 9120
caggatctcc tgtcatctca ccttgctcct gccgagaaag tatccatcat ggctgatgca 9180
atgcggcggc tgcatacgct tgatccggct acctgcccat tcgaccacca agcgaaacat 9240
cgcatcgagc gagcacgtac tcggatggaa gccggtcttg tcgatcagga tgatctggac 9300
gaagagcatc aggggctcgc gccagccgaa ctgttcgcca ggctcaaggc gcgcatgccc 9360
gacggcgagg atctcgtcgt gacccatggc gatgcctgct tgccgaatat catggtggaa 9420
aatggccgct tttctggatt catcgactgt ggccggctgg gtgtggcgga ccgctatcag 9480

gacatagcgt tggctacccg tgatattgct gaagagcttg gcggcgaatg ggctgaccgc 9540
ttcctcgtgc tttacggtat cgccgctccc gattcgcagc gcatcgcctt ctatcgcctt 9600
cttgacgagt tcttctgagg ggatctgata tcatcggatc ccccgggggc tgcaggtcga 9660
gggacctaat aacttcgtat aggatacttt atacgaagtt atattaaggg ttccggatcc 9720
tctagagtcg accccgggat gcagaattga tgatctatta acaataaaga tgtccactaa 9780
aatggaagtt tttcctgtca tactttgtta agaagggtga gaacagagta cctacatttt 9840
gaatggaagg attggagcta cgggggtggg ggtggggtgg gattagataa atgcctgctc 9900
tttactgaag gctctttact attgctttat gataatgttt catagttgga tatcataatt 9960
taaacaagca aaaccaaatt aagggccagc tcattcctcc cactcatgat ctatagatcc 10020
cccagcttca aaagcgctct gaagttccta tactttctag agaataggaa cttcggaata 10080
ggaacttcaa gatccactag cgataagctg cccccgacct cgacctgcag gcatgcaagc 10140
tagatagtgg gggttctcat gtaccacata cagagtggga tggttatatt gactgctgca 10200
ctggggctga cagagggcag ctgtctgtag ctgcttggtg gacagggcag gttaggaggg 10260
ctcctgattg ggatcactag gcactgcctt ctgctaagca gaggttgcct gggcctggaa 10320
gtgctagata attccaagga tgtaggaagg tgcacccaca aaacacaagt attgtaggca 10380
agggagaaga gggccatggt cccaggatgt ggtcttacct tagtgacttg cagggcattt 10440
tgggatccgg ctttgccctg aaagtccaag agcagcatcg gcaaaaacac tttgagaaac 10500
ggcggaaccc tgcggcaggt ctgatccagg tgagccttag tccctgttag ggaccaaccc 10560
ctgtcctgat ttcacctcac ccagaagtga acccagcagg atcctggcac tggcaatggc 10620
tgtaccatgc ttttccctgt atatggatag gctgtagcct gtgtttggaa aaacaaagtt 10680
gtgttcagca taagaaaaga ttatacatct gaggtgacac ctagtgactc atggtactgc 10740
tgctggatgg acctattctc agtggtcact tggctcaggt catactgtct caggaaccat 10800
ctaggttatt cccagtgtcc tgtgaggtaa gtgtctcatc acacctattc tgcagctgtg 10860
agacccacag cttagagaag ttgagtctga tagcaagagg ctcctgaggc acatctgtga 10920
cacttcggca tgagctgtgg acattgtgcc ttcctgcttt gcgcttgctc atgtggtctt 10980
tccattcagg ggcaggtgag gtgtgcaggc cactgaacat gcatggagaa gatagagatt 11040
cctcttgaac tctggagtta gccaggattt cctgccagat gaatgtggtc tctgtatctt 11100
tctttttaaa aaaaaaaaaa aaaaaagatg tggccttcac tgtcctaacc ttgggtccaa 11160
cttccttttg tgtctgtact gtatagcttt caccataaaa catatattgt tttgtaattt 11220
caaaatatac aaaaaggaac agattgccca tttgaaaagt aaaataattg catgtccaag 11280
gggccaaaag gtttaacagg gagcagcata aattgagtac cactcccata tatggaaaag 11340
ccattcaaca gtaccctgtg agtctagtac caccaaagag caatagcata gctgttcctg 11400
cctcatccac aagcccctcc ttatttgtct tgcctacagt ttccccactg tgaatttcag 11460
ggtggttccc agagcttcac agcacctcca acccatttat gatgcatgac cacagggtga 11520

aggtattgtg gtagaacaat tgatcaaaag gtcttccttt gaacaaagat gactttctac 11580
atatttgggg gcaaagcttt ggttaacaat gactccagta gcccccatgt aggtacaggg 11640
gtagaaaaac tgatttttca agaggattat cacctggtta tgattctggc actcaatcag 11700
aaaaatactt ggaggcagag tctcagagga ggtaccaggc ctttcatgcc ttcaaagtgt 11760
tctaaagaag tcagcctggc cggcctgtac cccatgtact ccttgatgtt attgaaagat 11820
tgatggagac caaagtaagt aaaaagaaat cccatgttca gggactggat gaaataatct 11880
cattcaaatg ttcttcccac acattggcat gcaggtccag tgccctccac ccccttcctg 11940
cggccgccac cgcggtggag ctccagcttt tgttcccttt agtgagggtt aattgcgcgc 12000
ttggcgtaat catggtcata gctgtttcct gtgtgaaatt gttatccgct cacaattcca 12060
cacaacatac gagccggaag cataaagtgt aaagcctggg gtgcctaatg agtgagctaa 12120
ctcacattaa ttgcgttgcg ctcactgccc gctttccagt cgggaaacct gtcgtgccag 12180
ctgcattaat gaatcggcca acgcgcgggg agaggcggtt tgcgtattgg gcgctcttcc 12240
gcttcctcgc tcactgactc gctgcgctcg gtcgttcggc tgcggcgagc ggtatcagct 12300
cactcaaagg cggtaatacg gttatccaca gaatcagggg ataacgcagg aaagaacatg 12360
tgagcaaaag gccagcaaaa ggccaggaac cgtaaaaagg ccgcgttgct ggcgtttttc 12420
cataggctcc gcccccctga cgagcatcac aaaaatcgac gctcaagtca gaggtggcga 12480
aacccgacag gactataaag ataccaggcg tttccccctg gaagctccct cgtgcgctct 12540
cctgttccga ccctgccgct taccggatac ctgtccgcct ttctcccttc gggaagcgtg 12600
gcgctttctc atagctcacg ctgtaggtat ctcagttcgg tgtaggtcgt tcgctccaag 12660
ctgggctgtg tgcacgaacc ccccgttcag cccgaccgct gcgccttatc cggtaactat 12720
cgtcttgagt ccaacccggt aagacacgac ttatcgccac tggcagcagc cactggtaac 12780
aggattagca gagcgaggta tgtaggcggt gctacagagt tcttgaagtg gtggcctaac 12840
tacggctaca ctagaaggac agtatttggt atctgcgctc tgctgaagcc agttaccttc 12900
ggaaaaagag ttggtagctc ttgatccggc aaacaaacca ccgctggtag cggtggtttt 12960
tttgtttgca agcagcagat tacgcgcaga aaaaaaggat ctcaagaaga tcctttgatc 13020
ttttctacgg ggtctgacgc tcagtggaac gaaaactcac gttaagggat tttggtcatg 13080
agattatcaa aaaggatctt cacctagatc cttttaaatt aaaaatgaag ttttaaatca 13140
atctaaagta tatatgagta aacttggtct gacagttacc aatgcttaat cagtgaggca 13200
cctatctcag cgatctgtct atttcgttca tccatagttg cctgactccc cgtcgtgtag 13260
ataactacga tacgggaggg cttaccatct ggccccagtg ctgcaatgat accgcgagac 13320
ccacgctcac cggctccaga tttatcagca ataaaccagc cagccggaag ggccgagcgc 13380
agaagtggtc ctgcaacttt atccgcctcc atccagtcta ttaattgttg ccgggaagct 13440
agagtaagta gttcgccagt taatagtttg cgcaacgttg ttgccattgc tacaggcatc 13500
gtggtgtcac gctcgtcgtt tggtatggct tcattcagct ccggttccca acgatcaagg 13560

cgagttacat gatcccccat gttgtgcaaa aaagcggtta gctccttcgg tcctccgatc 13620

gttgtcagaa gtaagttggc cgcagtgtta tcactcatgg ttatggcagc actgcataat 13680

tctcttactg tcatgccatc cgtaagatgc ttttctgtga ctggtgagta ctcaaccaag 13740

tcattctgag aatagtgtat gcggcgaccg agttgctctt gcccggcgtc aatacgggat 13800

aataccgcgc cacatagcag aactttaaaa gtgctcatca ttggaaaacg ttcttcgggg 13860

cgaaaactct caaggatctt accgctgttg agatccagtt cgatgtaacc cactcgtgca 13920

cccaactgat cttcagcatc ttttactttc accagcgttt ctgggtgagc aaaaacagga 13980

aggcaaaatg ccgcaaaaaa gggaataagg gcgacacgga aatgttgaat actcatactc 14040

ttcctttttc aatattattg aagcatttat cagggttatt gtctcatgag cggatacata 14100

tttgaatgta tttagaaaaa taaacaaata ggggttccgc gcacatttcc ccgaaaagtg 14160

ccac 14164

<210> 6
<211> 44
<212> DNA
<213> Artificial sequence

<220>
<223> Synthetic DNA (KQ2-LA5's)

<400> 6
gggaaggagc ggccgcagga agggggtgga gggcactgga cctg 44

<210> 7
<211> 45
<212> DNA
<213> Artificial sequence

<220>
<223> Synthetic DNA (KQ2-SA3'a)

<400> 7
gacggtgcgc ggccgccgtg gcagcctggg aaaggccaga aagat 45

<210> 8
<211> 38
<212> DNA
<213> Artificial sequence

<220>
<223> Synthetic DNA (lox71s)

<400> 8
ctagataact tcgtataatg tatgctatac gaacggta 38

<210> 9
<211> 38
<212> DNA
<213> Artificial sequence

<220>
<223> Synthetic DNA (lox71a)

<400> 9

ctagtaccgt tcgtatagca tacattatac gaagttat 38

<210> 10
<211> 32
<212> DNA
<213> Artificial sequence

<220>
<223> Synthetic DNA (Nh-loxPs)

<400> 10
tcgagctagc taataacttc gtatagcata ca 32

<210> 11
<211> 26
<212> DNA
<213> Artificial sequence

<220>
<223> Synthetic DNA (Nh-FRTa)

<400> 11
gaatgctagc ttgcatgcct gcaggt 26

<210> 12
<211> 4895
<212> DNA
<213> Plasmid

<400> 12
gtggcacttt tcggggaaat gtgcgcggaa cccctatttg tttatttttc taaatacatt 60

caaatatgta tccgctcatg agacaataac cctgataaat gcttcaataa tattgaaaaa 120

ggaagagtat gagtattcaa catttccgtg tcgcccttat tccctttttt gcggcatttt 180

gccttcctgt ttttgctcac ccagaaacgc tggtgaaagt aaaagatgct gaagatcagt 240

tgggtgcacg agtgggttac atcgaactgg atctcaacag cggtaagatc cttgagagtt 300

ttcgccccga agaacgtttt ccaatgatga gcacttttaa agttctgcta tgtggcgcgg 360

tattatcccg tattgacgcc gggcaagagc aactcggtcg ccgcatacac tattctcaga 420

atgacttggt tgagtactca ccagtcacag aaaagcatct tacggatggc atgacagtaa 480

gagaattatg cagtgctgcc ataaccatga gtgataacac tgcggccaac ttacttctga 540

caacgatcgg aggaccgaag gagctaaccg cttttttgca caacatgggg gatcatgtaa 600

ctcgccttga tcgttgggaa ccggagctga atgaagccat accaaacgac gagcgtgaca 660

ccacgatgcc tgtagcaatg gcaacaacgt tgcgcaaact attaactggc gaactactta 720

ctctagcttc ccggcaacaa ttaatagact ggatggaggc ggataaagtt gcaggaccac 780

ttctgcgctc ggcccttccg gctggctggt ttattgctga taaatctgga gccggtgagc 840

gtgggtctcg cggtatcatt gcagcactgg ggccagatgg taagccctcc cgtatcgtag 900

ttatctacac gacggggagt caggcaacta tggatgaacg aaatagacag atcgctgaga 960

taggtgcctc actgattaag cattggtaac tgtcagacca agtttactca tatatacttt 1020

agattgattt aaaacttcat ttttaattta aaaggatcta ggtgaagatc ctttttgata 1080

atctcatgac caaaatccct taacgtgagt tttcgttcca ctgagcgtca gaccccgtag 1140
aaaagatcaa aggatcttct tgagatcctt tttttctgcg cgtaatctgc tgcttgcaaa 1200
caaaaaaacc accgctacca gcggtggttt gtttgccgga tcaagagcta ccaactcttt 1260
ttccgaaggt aactggcttc agcagagcgc agataccaaa tactgtcctt ctagtgtagc 1320
cgtagttagg ccaccacttc aagaactctg tagcaccgcc tacatacctc gctctgctaa 1380
tcctgttacc agtggctgct gccagtggcg ataagtcgtg tcttaccggg ttggactcaa 1440
gacgatagtt accggataag gcgcagcggt cgggctgaac ggggggttcg tgcacacagc 1500
ccagcttgga gcgaacgacc tacaccgaac tgagatacct acagcgtgag ctatgagaaa 1560
gcgccacgct tcccgaaggg agaaaggcgg acaggtatcc ggtaagcggc agggtcggaa 1620
caggagagcg cacgagggag cttccagggg gaaacgcctg gtatctttat agtcctgtcg 1680
ggtttcgcca cctctgactt gagcgtcgat ttttgtgatg ctcgtcaggg gggcggagcc 1740
tatggaaaaa cgccagcaac gcggcctttt tacggttcct ggccttttgc tggccttttg 1800
ctcacatgtt ctttcctgcg ttatcccctg attctgtgga taaccgtatt accgcctttg 1860
agtgagctga taccgctcgc cgcagccgaa cgaccgagcg cagcgagtca gtgagcgagg 1920
aagcggaaga gcgcccaata cgcaaaccgc ctctccccgc gcgttggccg attcattaat 1980
gcagctggca cgacaggttt cccgactgga aagcgggcag tgagcgcaac gcaattaatg 2040
tgagttagct cactcattag gcaccccagg ctttacactt tatgcttccg gctcgtatgt 2100
tgtgtggaat tgtgagcgga taacaatttc acacaggaaa cagctatgac catgattacg 2160
ccaagcgcgc aattaaccct cactaaaggg aacaaaagct ggagctccac cgcggtggcg 2220
gccgctctag aactagtgga tccataactt cgtatagcat acattatacc ttgttatcta 2280
gtatggatga tgaaggaagg atgacctcct gtaggtacag ctgaagtctg aatgaccatc 2340
agtgtccagc ttcccctaaa ctacttggat gaacttccca tggctgagca gggacatctg 2400
gcctgggatt gcgacctctg tggtaggcca tttccagcct gcccaagtaa ccagagcccc 2460
ttacccctca gatcaccctg acgaccattg gctacgggga caagtaccct cagacctgga 2520
acgggaggct gctggcagcg acctttaccc tcattggtgt ctcgttcttt gctcttcctg 2580
ctgtgagtcc cgcgcacctt cctactctgg agatgttagg ggttttagag gctccccata 2640
aggcttggcc atggcccacc tgtgaactgt gaacttggac acagtcctgt ggggtttaca 2700
gaccctagag agctccagac tagaccagag tgaggctgct ctggtttaga tgcctgcggc 2760
ctccctaggc acacttctag tgtcttcaga tcaggccttc tcacatagct ctgttgattt 2820
ctgtggcttt cccatctgaa tgattctaag cttcaaaagc gctctgaagt tcctatactt 2880
tctagagaat aggaacttcg gaataggaac ttcaagatcc actagcgata agctgccccc 2940
gacctcgacg aattcatcga tgattcgacc tcgaaattct accgggtagg ggaggcgctt 3000
ttcccaaggc agtctggagc atgcgcttta gcagccccgc tgggcacttg gcgctacaca 3060
agtggcctct ggcctcgcac acattccaca tccaccggta ggcgccaacc ggctccgttc 3120

tttggtggcc ccttcgcgcc accttctact cctcccctag tcaggaagtt cccccccgcc 3180
ccgcagctcg cgtcgtgcag gacgtgacaa atggaagtag cacgtctcac tagtctcgtg 3240
cagatggaca gcaccgctga gcaatggaag cgggtaggcc tttggggcag cggccaatag 3300
cagctttgct ccttcgcttt ctgggctcag aggctgggaa ggggtgggtc cgggggcggg 3360
ctcaggggcg ggctcagggg cgggctcagg ggcggggcgg gcgcccgaag gtcctccgga 3420
ggcccggcat tctgcacgct tcaaaagcgc acgtctgccg cgctgttctc ctcttcctca 3480
tctccgggcc tttcgacctg catccatcta gatcagctta ccatgaccga gtacaagccc 3540
acggtgcgcc tcgccacccg cgacgacgtc cccagggccg tacgcaccct cgccgccgcg 3600
ttcgccgact accccgccac gcgccacacc gtcgatccgg accgccacat cgagcgggtc 3660
accgagctgc aagaactttt cctcacgcgc gtcgggctcg acatcggcaa ggtgtgggtc 3720
gcggacgacg gcgccgcggt ggcggtctgg accacgccgg agagcgtcga agcgggggcg 3780
gtgttcgccg agatcggccc gcgcatggcc gagttgagcg gttcccggct ggccgcgcag 3840
caacagatgg aaggcctcct ggcgccgcac cggcccaagg agcccgcgtg gttcctggcc 3900
accgtcggcg tctcgcccga ccaccagggc aagggtctgg gcagcgccgt cgtgctcccc 3960
ggagtggagg cggccgagcg cgccggggtg cccgccttcc tggagacctc cgcgccccgc 4020
aacctcccct tctacgagcg gctcggcttc accgtcaccg ccgacgtcga ggtgcccgaa 4080
ggaccgcgca cctggtgcat gacccgcaag cccggtgcct gacgcccgcc ccacgacccg 4140
cagcgcccga ccgaaaggag cgcacgaccc catgcatcgg ggggctgcag gtcgagggac 4200
ctaataactt cgtataggat actttatacg aagttatggt acccaattcg ccctatagtg 4260
agtcgtatta cgcgcgctca ctggccgtcg ttttacaacg tcgtgactgg gaaaaccctg 4320
gcgttaccca acttaatcgc cttgcagcac atcccccttt cgccagctgg cgtaatagcg 4380
aagaggcccg caccgatcgc ccttcccaac agttgcgcag cctgaatggc gaatgggacg 4440
cgccctgtag cggcgcatta agcgcggcgg gtgtggtggt tacgcgcagc gtgaccgcta 4500
cacttgccag cgccctagcg cccgctcctt tcgctttctt cccttccttt ctcgccacgt 4560
tcgccggctt tccccgtcaa gctctaaatc gggggctccc tttagggttc cgatttagtg 4620
ctttacggca cctcgacccc aaaaaacttg attagggtga tggttcacgt agtgggccat 4680
cgccctgata gacggttttt cgccctttga cgttggagtc cacgttcttt aatagtggac 4740
tcttgttcca aactggaaca acactcaacc ctatctcggt ctattctttt gatttataag 4800
ggattttgcc gatttcggcc tattggttaa aaaatgagct gatttaacaa aaatttaacg 4860
cgaattttaa caaaatatta acgcttacaa tttag 4895

<210> 13
<211> 32
<212> DNA
<213> Artificial sequence

<220>

<223>  Synthetic DNA (Hd-FRTs)

<400>  13
aacaagcttc aaaagcgctc tgaagttcct at                                    32


<210>  14
<211>  42
<212>  DNA
<213>  Artificial sequence

<220>
<223>  Synthetic DNA (Kp-lox2272a)

<400>  14
ataggtacca taacttcgta taaagtatcc tatacgaagt ta                         42


<210>  15
<211>  29
<212>  DNA
<213>  Artificial seqnece

<220>
<223>  Synthetic DNA (Neo3's)

<400>  15
gcaaaaccaa attccgggcc agctcattc                                        29


<210>  16
<211>  30
<212>  DNA
<213>  Artificial sequence

<220>
<223>  Synthetic DNA (KQ2CA3'a)

<400>  16
atttgtcctg cttcagtgct gtattgggat                                       30


<210>  17
<211>  26
<212>  DNA
<213>  Artificial sequence

<220>
<223>  Synthetic DNA (DT-A3's)

<400>  17
cctgtgcagg aaatcgtgtc agggcg                                           26


<210>  18
<211>  25
<212>  DNA
<213>  Artificial sequence

<220>
<223>  Synthetic DNA (KQ2LA5'a)

<400>  18
ttctcttcca gctggatcgg ggtgc                                            25


<210>  19
<211>  48

<212>  DNA
<213>  Artificial sequence

<220>
<223>  Synthetic DNA (BH-KMR/E6s)

<400>  19
taggatccat aacttcgtat agcatacatt ataccttgtt atctagta                    48


<210>  20
<211>  35
<212>  DNA
<213>  Artificial sequence

<220>
<223>  Synthetic DNA (Hd-E6a)

<400>  20
gataagctta gaatcattca gatgggaaag ccaca                                  35


<210>  21
<211>  31
<212>  DNA
<213>  Artificial sequence

<220>
<223>  Synthetic DNA (Y284Cs)

<400>  21
cgaccattgg ctacggggac aagtgccctc a                                      31


<210>  22
<211>  30
<212>  DNA
<213>  Artificial sequence

<220>
<223>  Synthetic DNA (Y284Ca)

<400>  22
gcctcccgtt ccaggtctga gggcacttgt                                        30


<210>  23
<211>  30
<212>  DNA
<213>  Artificial Sequence

<220>
<223>  Synthetic DNA (A306Ts)

<400>  23
ccctcattgg tgtctcgttc tttactcttc                                        30


<210>  24
<211>  30
<212>  DNA
<213>  Artificial Sequence

<220>
<223>  Synthetic DNA (A306Ta)

<400>  24
tcccaaaatg ccagcaggaa gagtaaagaa                                        30